# EUROPEAN PATENT SPECIFICATION

(11) **EP 1 696 953 B1**
(45) Date of publication and mention of the grant of the patent: **26.03.2014**
(21) Application number: 04820442.4
(22) Date of filing: 17.12.2004
(51) Int. Cl.: A61K 39/108, C12N 1/21

(54) **RECOMBINANT VIBRIO CHOLERAE STRAIN AND VACCINE COMPRISING SAID STRAIN**
REKOMBINANTER VIBRIO CHOLERAE STAMM UND IMPSTOFF, DER DIESEN STAMM ENTHÄLT
SOUCHE DE VIBRIO CHOLERAE RECOMBINEE ET VACCIN COMPRENANT CETTE SOUCHE

(30) Priority: 17.12.2003 EP 03029082
(43) Date of publication of application: 06.09.2006
(73) Proprietor: Crucell Switzerland AG, 3018 Bern (CH)
(72) Inventor: FAVRE, Didier, 3186 Düdingen (CH); DIETRICH, Guido, 3177 Laupen (CH); VIRET, Jean, François, 3177 Laupen (CH)
(74) Representative: Hateboer, Guus
(86) International application number: PCT/EP2004/014432
(87) International publication number: WO 2005/058354

(56) References cited:
- EP-A- 0 564 689
- WO-A-00/37106
- WO-A-94/01533
- WO-A-02/064162
- WO-A-03/022306
- WO-A-03/076643
- FAVRE D ET AL: "GENE REPLACEMENT IN GRAM-NEGATIVE BACTERIA: THE PMAKSAC VECTORS" BIOTECHNIQUES, EATON PUBLISHING, NATICK, US, vol. 28, no. 2, February 2000 (2000-02), page 198,200,202,204, XP001180140 ISSN: 0736-6205 cited in the application
- STEFFEN & DUPONT EDS.: "Textbook of travel medicine" 1999, MARCEL DEKKER , NY , XP001180710 Chapter 22.3 "Enteric vaccines: Present an future", by Levine & Svennerholm ISBN: 1550090372.m page 258, right-hand column, line 5 - page 260, left-hand column, last line
- KETLEY J M ET AL: "Construction of genetically marked Vibrio cholerae O1 vaccine strains" FEMS (FEDERATION OF EUROPEAN MICROBIOLOGICAL SOCIETIES) MICROBIOLOGY, vol. 111, no. 1, 1993, pages 15-21, XP002276310 1993 ISSN: 0378-1097 cited in the application
- CHEN I ET AL: "A RECOMBINANT LIVE ATTENUATED STRAIN OF VIBRIO CHOLERAE INDUCES IMMUNITY AGAINST TETANUS TOXIN AND BORDETELLA PERTUSSIS TRACHEAL COLONIZATION FACTOR" INFECTION AND IMMUNITY, AMERICAN SOCIETY FOR MICROBIOLOGY. WASHINGTON, US, vol. 66, no. 4, April 1998 (1998-04), pages 1648-1653, XP000946419 ISSN: 0019-9567
- DIETRICH G ET AL: "Experience with registered mucosal vaccines" VACCINE, BUTTERWORTH SCIENTIFIC. GUILDFORD, GB, vol. 21, no. 7-8, 30 January 2003 (2003-01-30), pages 678-683, XP004401604 ISSN: 0264-410X
- ACHESON D W K ET AL: "PROTECTIVE IMMUNITY TO SHIGA-LIKE TOXIN I FOLLOWING ORAL IMMUNIZATION WITH SHIGA-LIKE TOXIN I B-SUBUNIT-PRODUCING VIBRIO CHOLERAE CVD 103-HGR" INFECTION AND IMMUNITY, AMERICAN SOCIETY FOR MICROBIOLOGY. WASHINGTON, US, vol. 64, no. 1, January 1996 (1996-01), pages 355-357, XP001088560 ISSN: 0019-9567
- GAASTRA WIM ET AL: "Colonization factors of human enterotoxigenic Escherichia coli (ETEC)" TRENDS IN MICROBIOLOGY, vol. 4, no. 11, 1996, pages 444-452, XP002276311 ISSN: 0966-842X
- GALLEGOS MARIA-TRINIDAD ET AL: "AraC/XylS family of transcriptional regulators" MICROBIOLOGY AND MOLECULAR BIOLOGY REVIEWS, vol. 61, no. 4, December 1997 (1997-12), pages 393-410, XP002276312 ISSN: 1092-2172
- DATABASE BIOSIS [Online] BIOSCIENCES INFORMATION SERVICE, PHILADELPHIA, PA, US; 2003, PEI L ET AL: "Development of attenuated Vibrio cholerae vaccine vectors for inducing mucosal immune responses." XP002276313 Database accession no. PREV200300545984 & ABSTRACTS OF THE GENERAL MEETING OF THE AMERICAN SOCIETY FOR, vol. 103, 2003, pages E-018, 103rd American Society for Microbiology General Meeting;Washington, DC, USA; May 18-22, 2003, 2003 ISSN: 1060-2011 (ISSN print)
- DATABASE BIOSIS [Online] BIOSCIENCES INFORMATION SERVICE, PHILADELPHIA, PA, US; November 2003 (2003-11), ZHENG JI-PING ET AL: "Co-expression of CFA/I and CS6 of enterotoxigenic Escherichia coli (ETEC) in Shigella flexneri 2a T32 derivative strain FWL01." XP002276314 Database accession no. PREV200400076585 & SHENGWU HUAXUE YU SHENGWU WULI XUEBAO, vol. 35, no. 11, November 2003 (2003-11), pages 1005-1010, ISSN: 0582-9879 (ISSN print)

## Description

Gram-negative enteric pathogens are the cause of a variety of diseases presenting with a broad spectrum of symptoms ranging from mild watery diarrhea to severe life-threatening symptoms such as fever, bloody diarrhea, perforation or ulceration of the stomach or intestine, alone or in combination. Examples of such diseases include, but are not limited to, typhoid fever, shigellosis, cholera, infections with enterotoxigenic, enteropathogenic, and enterohemorrhagic *Escherichia coli,* and infections with *Helicobacter pylori* and *Campylobacter jejuni.*

The first step of the infectious process of enteric pathogens occurs at the mucosal surface within the digestive tract. Thus, interfering with this initial stage of infection prior to the onset of symptoms offers a particularly attractive approach for providing protection against enteric diseases. The most effective means by which to accomplish this would be to evoke a local protective immune response through the use of an orally administered vaccine (Walker, 1994). At present, two live oral attenuated vaccines against enteric disease have been licensed for human use, namely the Ty21 a strain of *Salmonella typhi* for the prevention of typhoid fever and the CVD 103-HgR strain of *Vibrio cholerae* for the prevention of cholera (Germanier and Fürer, 1975; Levine et al., 1988).

An effective protection against the enterotoxigenic *Escherichia coli* (ETEC) would be of particular medical interest. ETEC is one of the major causes of traveler's diarrhea and diarrhea among children in the developing countries and accounts for more than 1 billion diarrhea episodes and at least one million deaths per year, primarily among children.

ETEC isolates that cause diarrhea have several virulence factors that play important roles in the disease process. They include two enterotoxins, heat-labile toxin (LT) and heat-stable toxin (ST), as well as bacterial surface adhesins called pili or fimbriae which allow the organism to colonize the intestinal tract. Either one of the toxins can cause diarrhea. Some clinical ETEC isolates have been shown to produce either LT or ST, while other isolates have both toxins.

The most practical approach for the prevention of the widespread morbidity and mortality caused by diarrheal disease due to intestinal contamination with ETEC strains of *E. coli* would be by means of vaccination. The following three different types of *E. coli* antigen have been shown to be effective as immunogens which provide protection against challenge with ETEC strains in experimental models.
(i) Immunization of mice with either the *E*. *coli* LT holotoxin or its B subunit elicits an antitoxin response which provides protection against active challenge with the toxin itself and viable bacterial strains which produce either LT alone or both LT and ST toxins (Klipstein et al., 1981). However, immunizing with the holotoxin is not conceivable because of its toxicity to humans. On the other hand, the B subunit provides good immunogenicity and is non-toxic. However, since many ETEC strains produce only the ST toxin, an LT-B vaccine would only provide protection against a sub-group of ETEC infections from which all ST strains would be excluded. In contrast a combination of pili antigen and LT-B (or alternatively the cholera toxin B subunit [CT-B] which is highly homologous to LT-B) would be appropriate to protect against about 85% of all ETEC infections since it would cover many ST-producing strains.
(ii) Immunization with O-antigens prevents diarrhea by eliciting a mucosal immune response which helps reducing bacterial growth within the small intestine; this, however, extends only to homologous O-serotypes and not to heterologous O-serotypes. Since 170 antigenically dissimilar O-serotypes of *E*. *coli* including at least 78 for ETEC are known, immunization with O-antigens would require too many different vaccine strains to be effective (Wolf, 1997).
(iii) Immunization with fimbrial antigen (also known as pili or adhesins) responsible for adherence and colonization of the bacteria on the surface of the intestinal mucosa also provides protection. This protection, however, does not extend to ETEC strains possessing antigenically different fimbrial antigens (Morgan et al., 1978).

In addition to the above-mentioned *E*. *coli* immunogenic antigens, there is evidence that the B subunit of cholera toxin (CT-B) from *Vibrio cholerae* is also protective against ETEC strains expressing LT. This phenomenon may be explained by the similarity of CT-B and LT both in structure and in mode of action. Accordingly, cholera vaccines which include CT-B may be protective against ETEC diarrhea caused by ETEC isolates producing LT (Clemens et al., 1988). For example, Qadri et al. have recently shown that an oral vaccine containing a mixture of different ETEC pili antigens in combination with recombinantly produced cholera toxin B subunit (CT-B) is effective in children. In this phase II study carried out in Bangladesh, an oral vaccine comprising formalin-inactivated ETECs containing a total of six different surface antigens (CS 1-5 and CFA/I) were administered to children in combination with recombinant CT-B (Qadri et al, 2003). However, due to the use of killed cells, the level of immunogenicity afforded by this vaccine may not be optimal. As a consequence, two doses of the vaccine are necessary and the immunity may not be long-lasting. In contrast, live cells are thought to be more immunogenic, as they would more closely mimic a natural infection, also providing a longer-lasting immunity after only one dose (Levine and Kaper, 1993). Besides the fact that the inclusion of a purified toxin component raises the complexity and thus the cost of the vaccine, these considerations make it apparent that a live vaccine may be economically more viable.

An improvement with respect to this strategy would consist in delivering toxin-producing, pili-expressing live cells to the intestinal mucosa. Such a vaccine may be more efficiently sampled by the immune system due to its ability to penetrate the mucus layer overlaying the gut epithelium and adhere to the gut enterocytes. In turn, uptake by these and other specialized cells such as M cells with subsequent processing by antigen presenting cells would guarantee an optimal induction of a highly effective and specific mucosal immune response.

The induction of a local intestinal immune response may be the most efficient means by which to prevent infection with a number of enteric pathogens, including ETEC. A proven and effective method by which to accomplish this is through the use of live oral attenuated vaccine strains. Attenuated vaccine strains such as the above-mentioned *Salmonella typhi* Ty21a and *Vibrio cholerae* CVD 103-HgR elicit an abortive infectious process thereby inducing an immune response closely resembling that effected by natural infection. In addition, *V. cholerae* CVD 103-HgR has been shown to efficiently express a variety of foreign antigens in immunogenic form, among which the O-polysaccharide from *Shigella sonnei* and *V. cholerae* 0139, as well as shiga-like toxin Ib (Acheson et al 1993). Rabbits immunized with these constructs elicited significant antibodies directed against the heterologous antigen. Further, WO 94/19482 discloses a genetically engineered *V. cholerae* chromosome containing a heterologous DNA sequence encoding a bacterial antigen, wherein the DNA sequence is functionally linked to a naturally occurring *V. cholerae* promoter.

Both *Salmonella typhi* Ty21 a and *Vibrio cholerae* CVD 103-HgR possess the distinct advantage of being approved as being extremely safe in humans (Dietrich et al, 2003). Safety, however, has been found to be the most difficult attribute to achieve in the development of live oral vaccine strains. Most often, candidate vaccine strains either induce a protective immune response but with an unacceptable rate of adverse reactions or are safe but non-protective (Levine and Hone, 1991).

In earlier studies, pili of ETEC have been expressed in the *Shigella flexneri* 2a and *Salmonella typhimurium* vaccine strains (Altboum et al. 2001; Wu et al. 1995). However, these approaches suffer from the fact that the type of immunity elicited by these strains is quite different than that elicited by ETEC. This is primarily due to the infection mechanism of these strains. Whereas Shigella and Salmonella are invasive organisms that cross the gut epithelium and infect cells of the immune system, ETECs are non-invasive. The efficacy of these vaccine strains as ETEC vaccines may therefore be limited, since the presentation of the ETEC antigen(s) to the immune system would not mimic that existing in nature. In this respect, expression from a vaccine strain with virulence characteristics similar to those of ETECs would be highly advantageous.

ETEC itself has also been recently used as carrier for homologous and heterologous pili. However, although immunogenic, these strains present various problems. The pilis can only be efficiently produced in solid CF medium at a temperature of about 37°C. Furthermore, ETEC cells are known to be released by infected people for extended periods of time. Finally, the risk exists of stably acquiring an ETEC plasmid carrying the wild type LT and/or ST toxin genes by conjugation, thereby producing a virulent strain out of a vaccine strain. According to WO 03/022306, coli surface (CS) antigens may be expressed in ETEC strains. It is not clear, however, whether sufficient surface expression may be achieved.

The technical problem underlying the present invention is to develop a safe vaccine suitable to elicit an efficient immune response to a variety of ETEC isolates in human.

According to the present invention, this problem is solved by the provision of a recombinant *Vibrio cholerae* vaccine strain comprising DNA sequences coding for at least the ETEC pili CFA/I, CS3 and CS6, wherein the DNA sequences are operationally (functionally) linked to one or more promoters, and wherein the expression of one or more of said ETEC pili is enchanced by at least one ETEC regulator*.*

Since the virulence characteristics of wild-type *V. cholerae* are very similar to those of wild-type ETEC, the above-mentioned problems associated with the use of vaccine strains such as *Shigella* and *Salmonella* for the expression of ETEC adhesins are overcome by the recombinant *V. cholerae* strain according to the present invention. Both *V. cholerae* and ETEC colonize the small intestine without crossing the gut epithelium and secrete powerful toxins which mediate disease by causing net secretion into the intestinal lumen. The cholera toxin (CT) and the ETEC toxin (LT) share a homology of about 83% on the amino acid level and have an identical AB₅ structure. Most importantly, both pathogens elicit an immune response in a similar way. They are sampled by the immune system via the M cells of the Peyer's patches and elicit a local intestinal immune response through mucosal sIgA which can clear subsequent infection by immune exclusion.

The term "DNA sequence" refers to a nucleic acid sequence encoding the primary sequence of a protein. The term "ETEC pilus" as used herein refers to a polypeptide consisting essentially of, and having a similar immunological activity as the polypeptides listed in table 1. The immunological activity may be determined by parenteral immunization of mice and is considered as being "similar" if the subsequent detection of specific anti-pili antibodies in an enzyme-linked immunosorbent assay (ELISA) provides a significant titer. A significant ELISA titer is defined as being equal or superior to twice the reciprocal of the background ELISA titer.

The definition of "ETEC pilus" intended to encompass polypeptides comprising natural allelic variations, orthologs, artificially constructed variants, and derivatives of the pili listed in table 1. The term "ortholog" refers to a polypeptide from another species that corresponds to the human ETEC polypeptides as listed below. The term "variant" refers to a polypeptide comprising one or more amino acid substitution(s), deletion(s) and/or addition(s) as compared to the polypeptides as set forth in table 1. The term "derivative" refers to polypeptide allelic or artificially constructed variants and orthologs, as defined herein, that have been chemically modified.

**Table1: ETEC pili types**

| Pili type^{a} | Genes | Genetic location and notes | Reference |
|---|---|---|---|
| CFA/I | *cfaA, B, C, E* | plasmid | Gaastra and Svennerholm, 1996 |
| CS1 (CFA/II) | *coo or csoA, B, C, D* | Plasmid | Marron et al, 1995 |
| CS2 (CFA/II) | *cotA, B, C, D* | Chromosome | Froehlich et al, 1995 |
| CS3 (CFA/II) | *cstA, B, C* | plasmid, cstB contains 5 overlapping ORF | Gaastra and Svennerholm, 1996 |
| CFA/III (CS8) | *cofA* | plasmid | Taniguchi et al, 1995 |
| CS4 (CFA/IV) | *csaA, B, C, E, D* | Plasmid | Altboum et al, 2003 |
| CS5 (CFA/IV) | *csfA, B, C, E, F, D* | Plasmid | Duthy TG et al, 1999 |
| CS6 (CFA/IV) | *cssA, B, C, D* | Plasmid | Gaastra and Svennerholm, 1996 |
| CS7 | | ? | Gaastra and Svennerholm, 1996 |
| CS10 (2230) | | Plasmid | Darfeuille-Michaud A. et al 1986 |
| CS11 (PCFO148) | | ? | Knutton S. et al, 1987 |
| CS12 (PCFO159) | | ? | Schmidt H et al., 1995 |
| CS13 (PCFO9) | | ? | Heuzenroeder MW et al, 1990 |
| CS14 (PCFO166) | *csuA₁, A₂, B, C, E* | ? | Gaastra and Svennerholm, 1996 |
| CS15 (8786) | | Plasmid | Aubel et al, 1991 |
| CS17 | | ? | McConnell et al, 1990 |
| CS18 (PCFO20) | *fotA,B,C,D,E,F,G* | Chromosome (?) | Viboud et al, 1996 |
| CS19 | | ? | Grewal et al, 1997 |
| CS20 | | ? | Valvatne et al, 1996 |
| CS21 (Longus) | *IngA* | ? | Giron JA et al, 1997 |
| CS22 | *cseA* | ? | Pichel et al, 2000 |

| | | | |
|---|---|---|---|
| *^{a} In parentheses: previous denomination* | | | |

In humans, 21 different pili types have been described (see table 1 above), some of which occur together in the same organism. One pilus type, coli surface antigen 3 (CS3), is detected in 23% of ETEC isolates and occurs either alone or in combination with CS1 or CS2. CS6, detected in 21% of ETEC isolates, occurs either alone or in combination with CS4 or CS5. Colonization factor I (CFA/I) occurs alone in about one third of all ETEC isolates. These data are consistent with the idea that a potential vaccine comprising CFA/I, CS3 and CS6 may provide protection against a maximum of about 75% of all ETEC cases.

The *V*. *cholerae* strain according to the present invention may comprise further DNA sequence(s) encoding ETEC pili. In a preferred embodiment, the DNA sequence comprised in the *V. cholerae* strain according to the invention is selected from the group consisting of the sequences encoding the 21 pill described in table 1. Up to nine additional sequences coding for an ETEC pilus may be contained. Preferably, the strain of the invention comprises at least one further DNA sequence. More preferably, at least two or three further sequences are comprised. Optionally, pill from other pathogenic *E. coli* such as EaggEC (AAF/I pili), EHEC, EPEC (BFP, AF/R1), or veterinary ETEC (K88, K99, 987P), as well as from other bacteria may be Included.

In a preferred embodiment, expression of the ETEC pili is driven by one or more ETEC promoter(s) (e.g. by the promoter naturally occuring in respective operon). Alternatively, an ETEC promoter from an heterologous pill operon, or promoter(s) from a source other than ETEC may be used (e.g. the lambda phage P_{L} promoter [Schauder B et al., 1987] or the Ptrc promoter [Amann E et al., 1988]),

Genetically, the formation of CFA/I, CS3 and CS6 pill is encoded in operons comprising 4-7 genes each. Thus, in a preferred embodiment, the DNA sequence encoding the ETEC pilus comprises the entire operon.

In addition, the expression of many pili is up-regulated through the translational product of an additional gene which is encoded by a DNA sequence lying outside the respective operon. They are *cfaD* for CFA/I, *rns* for CS1 and CS2, and *csvR* for CS4. These regulatory genes show homology at the DNA level and were found to be functionally interchangeable (table 2).

**Table 2: Regulators**

| **Genes** | **Pili regulated** | **Genetic location** | **Reference** |
|---|---|---|---|
| *cfaD* | CFA/I | plasmid | Savelkoul et al, 1990 |
| *csvR* | CS4, CS5 | plasmid | de Haan et al, 1991 |
| | | | |
| *ms* | CS1, CS2 | plasmid | Caron at al, 1989 |
| *aggR* | AAF/I | | Cassels and Wolf, 1995 |

In contrast to the expression of CFA/I, CS1, CS2 and CS4 in ETEC, which is enhanced by a regulator, expression of CS3 and CS6 is not enhanced in ETEC cells (Caron et al, 1990). That is, CS3 and CS6 expression obviously occurs independently from any regulation in their natural ETEC host. CFA/I expression is up-regulated by the *cfaD* gene product in ETEC. However, heterologous expression of CFA/I carried on a plasmid is known to occur without the need of a regulator in a foreign host such as a laboratory *E. coli* strain and *Salmonella typhimurium* (Wu et al. 1995).

Surprisingly in view of the above, the present authors found that expression of CFA/I and CS3 pili in a *V. cholerae* recipient strain was very poor even under the media and temperature conditions of growth that are normally known to lead to optimal pill synthesis. In contrast, the expression of CS6 pili was as good as in the ETEC wild-type strain B7A and thus considered satisfactory.

in order to improve the heterologous expression of CFA/I and CS3 in *V. cholerae,* expression of the CFA/I, CS3, and CS6 pili in the presence of the ETEC regulators was analyzed. For that purpose, the gene for the Rns regulator was chromosomally integrated in strains carrying either the CFA/I or the CS3 locus. Expression of the ETEC regulator resulted in an increased expression of the structural pili proteins which translated into the surface formation of pill (which could be detected by various means including electron microscopy). Both CS3 and CFA/I expression was highly increased by Rns (see example 7 and 9). This was surprising since these operons are not supposed to be regulated inside ETEC or in a foreign host, respectively (see above). The expression of CS6 pilus was not increased by any of the regulators.

In a preferred embodiment of the present invention, expression of the ETEC pill is therefore enhanced by at least one ETEC regulator. The regulator may be selected from the group consisting of Rns, CfaD, CsvR and AggR. In another embodiment of the present invention the regulator is Rns.

The term "regulator" refers to the translational product (polypeptide) encoded by any of the genes listed in table 2, as well as naturally occurring variations, orthologs, artificially constructed variants, and derivatives thereof.

In one embodiment of the present invention a recombinant *V*. *cholerae* strain is obtained by introducing the DNA sequences into a recipient strain selected from the group consisting of CVD 103-HgR, CVD111, CVD112 and Peru-15. CVD 103-HgR or an equivalent strain is particularly preferred. Like CVD 103-HgR, equivalent strains show inactivation of the *ctxA* gene. Inactivation may preferably be achieved by deletion.

The term "recipient strain" refers to the strain which has been transformed, or is capable of being transformed with a DNA sequence and then of expressing a selected gene of interest. The progeny of said strain containing the selected DNA sequence is referred to as the recombinant strain. The term "parent strain" refers to the strain upstream of any given strain in the respective strain history.

CVD 103-HgR is a live oral attenuated *V. cholerae* vaccine strain which originates from *V*. *cholerae* classical Inaba strain 569B and which has 94 % of the ctxA DNA sequence deleted but still expresses the immunogenic B subunit of CT (Ketley et al., 1993). As stressed above, expression of the ETEC adhesins from a live oral attenuated *V. cholerae* vaccine strain such as CVD 103-HgR is advantageous, since the virulence characteristics of wild-type *V*. *cholerae* are very similar to those of wild-type ETEC. Other *V*. *cholerae* potential live vaccine strains which may be useful for the expression of ETEC pilis are CVD111 (Taylor et al, 1999) and Peru-15 (Kenner et al, 1995), both derived from EI Tor O1 strains, and CVD112 (Tacket et al, 1995) and Bengal-15 (Coster et al, 1995), both derived from strains of the 0139 serotype. Advantage of use of the above strains stem from the fact that i) *V*. *cholerae* EI Tor is the only biotype currently isolated worldwide and ii) 0139 is the only non-01 serotype causing disease in humans.

In one embodiment of the invention, the DNA sequences coding for the pili are either present on a plasmid or stably integrated into the chromosome of the recipient strain.

The term "plasmid" is used to refer to a molecule capable of autonomous replication that is suitable for transformation of a recipient strain and contains DNA sequences that direct and/or control the expression of inserted heterologous DNA sequences. Various types of plasmids may be used such as low and high copy number plasmids, narrow and broad-host range plasmids, expression plasmids, and cosmids. High copy number plasmids are preferred, provided that they are not toxic to the cells. The latter seems to be case for the CS3 operon, for example. In these cases, low-copy number plasmids are preferred.

In general, heterologous gene expression is achieved by cloning of the heterologous gene(s) into plasmids which are replicated within the recipient in multiple copies thus leading to high expression of foreign gene product. However, synthetic plasmids are notably unstable, being rapidly lost from the cells when the latter are grown without selection pressure, giving rise to a cell population which does not express the heterologous antigen any longer. A further risk is that of plasmid(s) transfer to other bacterial species either in the environment or in the gut. Although some plasmids have been developed which address the first concern, the safety aspect remains a potential concern. Taken together, the expression of foreign antigens from plasmids is not appreciated by the regulatory authorities. Accordingly, the expression of foreign antigens is preferably achieved from corresponding genes integrated into the chromosome of the recipient strain, where they are stably maintained, while the risk of unwanted transfer to other organisms is excluded.

In order to circumvent both stability and safety concerns, the *cfa, cst,* and *css* loci encoding the CFA/I, CS3, and CS6 pili, respectively, preferentially are integrated into the chromosome of the *V. cholerae* CVD 103-HgR recipient strain. The co-integration of 2-3 operons per one strain is preferred. In a preferred embodiment, the DNA sequence encoding the ETEC pilus is chromosomally integrated into an integration site which is non-essential for inducing a protective immune response by the strain, such as the *hlyA, hlyB, rtxA* and/or *orfU* genes, or any gene within the ctxφ region of *V. cholerae.* The *hlyB* locus is particularly preferred for integration.

Similarly, the DNA sequence encoding the regulator can either be present on a plasmid or stably integrated into the chromosome of the recipient strain. In a preferred embodiment, the DNA sequence encoding the ETEC regulator is chromosomally integrated into the *hlyA, hlyB, rtxA* and/or *orfU* genes, or any gene within the ctxφ region of *V. cholerae.* The *hlyB* locus is particularly preferred for integration.

In a further embodiment of the invention, multiple copies of the DNA sequence encoding the pilus and/or regulator are integrated in tandem into a single chromosomal integration site or independently integrated into individual integration sites. Preferably, 1 to 10 copies of the DNA sequence encoding the pilus and 1 to 10 copies of the DNA sequence encoding the regulator are inserted, more preferably 1-2 copies.

In vitro as well as in vivo, the expression of pili is temperature-dependent and occurs at around 37°C, usually less well at lower temperatures (Cassels and Wolf, 1995). It was therefore surprising that it proved to be possible to obtain the same level of expression after growth of the recombinant strains at 30 and 37°C. In a preferred embodiment of the invention, expression of the ETEC pili therefore occurs during growth in liquid medium at a temperature between 25°C and 42°C. Expression during growth at a temperature of 37°C is particularly preferred.

In one embodiment of the invention, the recombinant *V.cholerae* strain is characterized in that the strain has retained the characteristics of the recipient strain with respect to LPS and CT subunit B production, and cytotoxicity.

*V. cholerae* O1 Inaba lipopolysaccharide (LPS) may be identified by Western blotting with the specific monoclonal antibody VCO4 (Gustafsson and Holme, 1983). CT-B production can be measured by the GM-1 ELISA assay as described (Svennerholm and Holmgren, 1978). A positive production of CT-B is defined by a value of more than 2 µg/ml. Cytotoxicity may be determined by the Y1 adrenal assay as described (Sack and Sack, 1975) and is defined as a rounding of Y1 adrenal cells

In a preferred embodiment, the strain shows increased adherence to gut epithelial cells as compared to the recipient strain. The adherence may be determined by assessing the capacity of a cell expressing the respective polypeptide to adhere to gut cells. The adherence as defined in the present invention is considered to be "similar", if the normalized adherence index NAI is in the range of 0.9-1.1 if compared to that of CVD 103-HgR (for details, see example 11); the adherence is considered to be "increased", if the normalized adherence index NAI is >1.

The capacity of the strain to adhere to gut cells may be determined as outlined in example 11 of the present description. Accordingly, an NAI above 1 indicates higher adherence than that of CDV1 03-HgR.

In another aspect, the present invention is related to a vaccine comprising the recombinant *V. cholerae* strain according to the invention, optionally in a mixture with a pharmaceutically acceptable excipient (e.g. lactose and/or ascorbic acid) and/or a buffer (e.g. sodium bicarbonate). In a preferred embodiment, the vaccine may further comprise other bacterial strains and/or toxins. Examples for toxins are given in the table below. A vaccine in which co-expression of 01 cholera LPS, the cholera toxin B-subunit and the ETEC pili is achieved may result in an enhanced protection rate and thus could resemble an alternative vaccine composition protective not only against ETEC, but also against cholera. By inclusion of a CT-B toxin, it may be possible to increase the protection rate to 85% of all ETEC isolates.

### TOXINS

| Genes | Toxins | | Reference |
|---|---|---|---|
| eltAB | LT-A, LT-B | heat-labile enterotoxin, plasmid | Sears and Kaper, 1996 |
| sta | ST-A, ST-B | heat-stable enterotoxin, plasmid | Sears and Kaper, 1996 |
| east1 | EAST-1 | plasmid/chromosome | Yamamoto and Echeverria, 1996 |

In a further aspect, the present invention relates to the use of the recombinant *V. cholerae* strain according to the invention for preparing a pharmaceutical composition for immunization against ETEC and/or cholera.

Preferably, the pharmaceutical composition is for mucosal administration, most preferred is oral, nasal, and/or rectal administration.

In yet another aspect, the present invention is related to a method for producing the recombinant *V. cholerae* strain according to the invention, said method comprising the steps of
(a) introducing the DNA sequences coding for at least the ETEC pili CFA/I, CS3 and CS6 into a strain according to claim 8 and, optionally,
(b) further adding one or more of the DNA sequences coding for an ETEC regulator selected from the group consisting of csfR, rns, cfaD, csvR and aggR.

Further, the present invention also relates to a method for preparing a vaccine composition, comprising formulating the recombinant *V. cholerae* strain according to the invention in a mixture with a pharmaceutically acceptable excipient and/or buffer.

In another aspect, the present invention is related to methods for increasing the immunogenicity of a *V. cholerae* strain by increasing its adherence to the gut cells.

This may be achieved by introducing at least one DNA sequence encoding an ETEC pilus into the *V. cholerae* strain and culturing said strain under conditions which allow surface expression of the pili. A DNA sequence encoding CS3 is particularly preferred. Thus, ETEC pili are expressed on the surface of the *V. cholerae* cells in addition to homologous *V*. *cholerae* pili. In a preferred embodiment, at least one DNA sequence encoding a regulator gene is further introduced into the strain.

Certain *V. cholerae* strains, such as CVD 103-HgR, are known to present an impaired adherence compared to their parent strains, e.g. CVD 103 (Ketley et al., 1993). Furthermore, various studies suggest that some genetic modifications (Ketley et al., 1990), as well as the expression of foreign antigens in *V. cholerae* may reduce colonization with respect to the parent strain. In one study, for example, the addition of the *eaeA* gene of EHEC which codes for an outer membrane protein suppressed the immune response against the cloned subunit B of Shiga toxin. This effect may be due to lower growth and/or colonization by the *eaeA*-expressing strain (Butterton et al., 1997). In summary, the expression of a foreign antigen in a *V. cholerae* strain such as CVD 103-HgR may adversely influence the adherence properties of said strain. This, in turn, may negatively influence the magnitude of the immune response elicited by the recombinant strain, since the induction of an efficacious immune response against *V*. *cholerae* is linked to the ability of the cells to adhere to the gut cells in order to i) express the CT toxin in vivo, and ii) to be sampled by the immune system at the Peyer's patches. It is therefore indispensable to guarantee the adherence of the recombinant strain. This may be achieved by expressing further adherence determinants in the recombinant strain, thereby increasing its adherence properties.

In the present invention, it was examined whether an increased adherence of CVD 103-HgR could be achieved by expression of the ETEC pili in the *V. cholerae* strain. For that purpose, the adherence properties of the recombinant strain were compared to those of the recipient CVD 103-HgR strain. It was found that, indeed, the adherence of the parent strain can be increased by this strategy (see example 11).

According to the invention, the surface expression of at least one ETEC pilus in a *V*. *cholerae* strain may further be enhanced by co-expression of a regulator gene which is stably integrated into the chromosome of the strain or present on a plasmid (see examples 7 and 9). For that purpose, at least one DNA sequence encoding a regulator gene is introduced into said strain and said strain is then cultured under conditions which allow expression of the pilus and the regulator, wherein the regulator gene is stably integrating into the chromosome of the strain or present on a plasmid. Preferentially, expression of CFA/I and/or CS3 may be enhanced using said method.

The following Figures and examples shall illustrate the present invention in more detail. The examples given shall not be taken to limit the scope of the invention.

### Figures

Fig.1: Construction of pili and regulator integration vectors. The arrows inside the plasmids represent the direction of transcription of the genes.
Fig.2: Construction of the CS3 operon integration plasmid pMAKCS3-2.
Fig.3: Construction of the CS6 operon integration plasmid pMAKCS6-1.
Fig.4: Construction of the CFA/I integration plasmid pMAKcfalS-4.
Fig 5: Integration of the regulator *csfR* or *rns* genes in the *hlyB* gene
Fig.6: Western Blot of the expression of CS3 pilin in CVD 103-HgR. The CS3 pilin was identified by use of a polyclonal CS3-specific rabbit antiserum. Lanes: 1. Wild-type ETEC DS7-3; 2. DH10B (pSSVI215); 3. DH10B (pSSVI215-CS3); 4. DH10B (pGB1); 5. CVD 103-HgR (pSSVI215); 6. CVD 103-HgR (pSSVI215-CS3); 7. CVD 103-HgR (pSSVI215-CS3/pM392); 8. CVD 103-HgR (pGB1); 9. CHCS3-2 colony #1; 10. CHCS3-2 colony #2; 11. CHCS3-2 #1 (pM392), 30°C.; 12. CHCS3-2 #1 (pM392), 37°C.; 13. CHCS3-2 #2 (pM392), 30°C.; 14. CHCS3-2 #2 (pM392), 37°C.
Fig.7: Western Blot analysis of the expression of CS6 pilin in CVD 103-HgR. The CS6 pilin was identified by use of a polyclonal CS6-specific rabbit antiserum. Lanes: 1. ETEC B7A; 2. CVD 103-HgR (pM295), 30°C; 3. CVD 103-HgR (pM295), 37°C; 4. CVD 103-HgR (pSSVI215); 5. CVD 103-HgR (pSSVI215-CS6); 6. CVD 103-HgR (pSSVI215-CS6/pM392), 30°C; 7. CVD 103-HgR (pSSVI215-CS6/pM392), 37°C; 8. CHCS6-1; 9. CHCS6-1 (pM392); 10. CHCS6-2 (pM392); 11. CVD 103-HgR (pSSVI215).
Fig.8: Western Blot analysis of the expression of CFA/I pilin in CVD 103-HgR. The CFA/I pilin was identified by use of a polyclonal CFA/I-specific rabbit antiserum. Lanes: 1. Wild-type ETEC H10407; 2. CVD 103-HgR (pSSVI215), 3. CVD 103-HgR (pSSVI215-cfaI); 4. CVD 103-HgR (pSSVI215-cfaI/pM392), 30°C; 5. CVD 103-HgR (pSSVI215-cfaI/pM392), 37°C; 6. CHcfal-1; 7. CHcfal-1 (pM392), 30°C; 8. CHcfal-1 (pM392), 37°C; 9. CHcfal-2; 10. CHcfaI-1 (pM392), 30°C; 11. CHcfal-2 (pM392), 37°C; 12. CVD 103-HgR (pK18cfaI-a).
Fig.9: Western blot analysis of pilin expression in the presence of either the CsfR or the Rns regulator. Fig.4A (CFA/I), lanes: 1.ETEC H10407 2.CHcfal-1 3.CHcfal-2; 4.CHcfaI-1 (pCLrns); 5.CHcfal-2 (pCLrns); 6.CHcfal-1 (pEU2040); 7.CHcfal-2 (pEU2040); 8.CHcfaI-1 (pM392); 9.CHcfal-2 (pM392); Fig.4B (CS3), lanes: 1.DS7-3; 2.CHCS3-2; 3.CHCS3-2 (pCLrns); 4.CHCS3-2 (pEU2040); 5.CHCS3-2 (pM392); Fig.4C (CS6), lanes: 1.ETEC B7A. 2.CHCS6-1; 3.CHCS6-2; 4.CHCS6-1 (pCLrns); 5.CHCS6-2 (pCLrns); 6.CHCS6-1 (pEU2040) 7.CHCS6-2 (pEU2040) 8.CHCS6-1 (pM392).

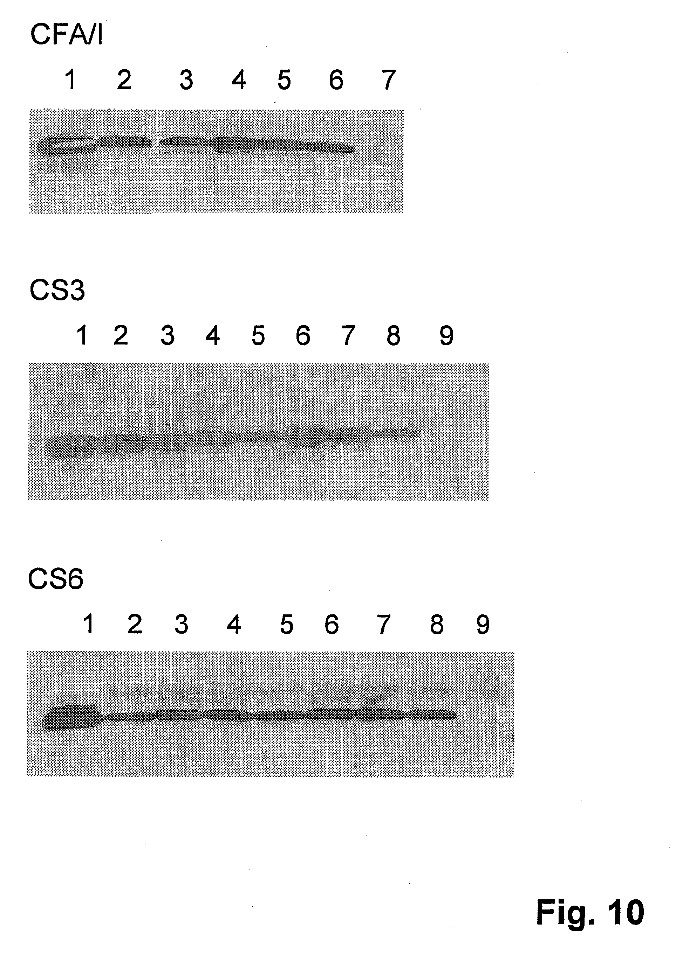

Fig.10: Western blot analysis of pili expression in recombinant CVD 103-HgR/ETEC strains grown in liquid cultures at 37°C. Fig.10A(CFA/I), lanes: 1.ETEC H10407 grown on solid CF; 2.CHcfal-2 (pM392) grown on solid CF; 3.CHcfal-2 (pM392), 16 h in liquid CF; 4.CHcfal-2 (pM392) in liquid CF collected at OD₆₀₀= 0.5; 5.CHcfal-2 (pM392) in liquid CF collected at OD₆₀₀= 1.5; 6.CHcfal-2 (pM392) in liquid CF collected after 16 h incubation 7.CVD 103-HgR grown on solid CF. Fig.10B (CS3), lanes: 1.ETEC DS7-3 grown on solid CF 2.CHCS3-2 (pM392) #1 grown on solid CF; 3.CHCS3-2 (pM392) #1 collected at OD₆₀₀ = 0.5; 4.CHCS3-2 #1 (pM392) CF collected at OD₆₀₀= 1.5: 5.CHCS3-2 (pM392) in liquid CF collected after 16 h incubation; 6.CHCS3-2 (pM392) #2 collected at OD₆₀₀ = 0.5; 7.CHCS3-2 #2(pM392) CF collected at OD₆₀₀= 1.5: 8.CHCS3-2 (pM392) #2 in liquid CF collected after 16 h incubation; 9.CVD 103-HgR grown on solid CF. Fig.10C (CS6), lanes: 1.ETEC B7A grown on solid CF 2.CHCS6-1 grown on solid CF; 3.CHCS6-1 in liquid CF, pH 6.5 collected at OD₆₀₀ = 0.5; 4.CHCS6-1 grown in liquid CF, pH 6.5 collected at OD₆₀₀= 1.5: 5.CHCS6-1 in liquid CF, pH 6.5 collected after 16 h incubation; 6.CHCS6-1 in liquid CF, pH 7.3 collected at OD₆₀₀ = 0.5; 7.CHCS6-1 grown in liquid CF, pH 7.3 collected at OD₆₀₀= 1.5: 8.CHCS6-1 in liquid CF, pH 7.3 collected after 16 h incubation; 9.CVD 103-HgR grown on solid CF.
Fig. 11 Western blot analysis of pilin expression in recombinant strains containing an integrated *rns* regulator gene. Panel CFA/I: A. Lanes: 1. H10407 ETEC; 2. CHcfal-1; 3. CHcfal-1(pMAKSAChlyAR/rnsOri1); 4. CHcfaI-R1; 5. CVD 103-HgR. B: Lanes: 1. Molecular weight markers; 2. CVD 103-HgR; 3. BB01; 4. CD79a. Panel CS3: Lanes: 1. DS7-3, 37°C; 2. CHCS3-2, 37°C; 3. CHCS3-2 (pEU2040), 37°C; 4. CHCS3-2(pMAKSAChlyAR/rnsOri2), 30°C; 5. CHCS3-R2 #1, 37°C; 6. CHCS3-R2 #1, 30°C; 7. CHCS3-R2 #2; 37°C; 8. CHCS3-R2 #2, 30°C; 9. CVD 103-HgR, 37°C
Fig.12: Immunofluorescence assay of the expression of ETEC pilis in CVD 103-HgR. Panel A: top: CVD 103-HgR; bottom: CHCFA-1 (pM392). Panel B: top: CVD 103-HgR, bottom: CHCS3-2 (pM392); Panel C: top: CVD 103-HgR, bottom: CHCS6-2.
Fig.13: Ultrastructure of recombinant *V. cholerae-*ETEC strains by scanning electron microscopy. Panel A: top: ETEC H10407 (produces CFA/I pilis); bottom: CHCFA-1 (pM392); Panel B: top: ETEC DS7-3 (produces CS3 pilis), bottom: CHCS3-2 (pM392); Panel C: top: ETEC B7A (produces CS6 pilis), bottom: CHCS6-2; Panel D: CVD 103-HgR.
Fig. 14: Synthesis of CFA/I pili by monovalent CFA/I strain BB01 and bivalent CFA/I-CS3 strain BB06.
Fig. 15: LPS analysis of recombinant CVD 103-HgR/ETEC strains. Panel A: Silver stained SDS-polyacrylamide gel. Panel B: Western blot probed with the *V.cholerae* O1 Inaba-specific Mab VCO4. Lanes: 1.Molecular weight standards. 2.CVD 103-HgR 3.B7A. 4.CHCS6-1. 5.CHCS6-2. 6.DS7-3. 7.CHCS3-R2 #1 8.CHCS3-R2 #2 9.H10407. 10.CHcfa1-R1 # 2.
Fig. 16: Detection of CS6, CS3, and CFA/I pilins in trivalent vaccine strains. Lanes: 1. CD79a; 2. B7A; 3. DS7-3; 4. CVD 103-HgR; 5. BB06 (pSSVI215-CS6); 6. BB07 (pSSVI215-CS6); 7. Panel CS6: CVD 103-HgR (pSSVI215-CS6), Panel CS3: CHCS3-R2, Panel CFA/I: BB01.
Fig.17: Genetic map of the inserted pili and regulator loci in the *V. cholerae* bivalent strains BB06 and BB07

### Examples

### 1. Cultivation of strains

The bacterial strains and plasmids used in this study are listed in Table 3.

**Table 3: Strains and plasmids**

| Strains | Genotype | Source or Reference |
|---|---|---|
| E.coli K12 | | |
| DH1OB | *araD139, Δ(ara-leu7697), galU, galK, mcrA, Δ(mrr-hsdRMS-mcrBC), rpsL, deoR, Φ 80dlacZΔM15, endA1, nupG, recA1.* | |
| S17.1 | *thi-1 pro hsdR Tp^{r} Sm^{r} RP4-2 [Tc::Mu (Km:: Tn7)]* | Simon et al, |
| | | |

| Enterotoxigenic E.coli (ETEC) | | |
|---|---|---|
| H10407 | *cfaABCE, LT, ST* | W.Gaastra |
| CD79a | *cfaABCE, LT, ST* | J. Blanco |
| DS7-3 | *cstABC* | M.K. Wolf |
| B7A | *cssABCD* | M.K. Wolf |
| | | |

| V. cholerae | | |
|---|---|---|
| CVD 103-HgR | *ΔctxA hlyA::mer (Hg^{R})* | Ketley et al, 1993 |
| CHcfaI-1 | CVD 103-HgR *hlyB::cfaIABCE,* HlyB→cfaI→ | this work |
| CHcfaI-2 | CVD 103-HgR *hlyB::cfaIABCE,* HlyB→cfaI← | this work |
| CHcfaI-4 | CVD 103-HgR *hlyB::cfaIABCE,* HlyB→cfaI→ | this work |
| CHCS3-2 | CVD 103-HgR *hlyB::cstABC* HlyB→cst← | this work |
| CHCS6-1 | CVD 103-HgR *hlyB::cssABCD,* HlyB→css→ | this work |
| CHCS6-2 | CVD 103-HgR *hlyB::cssABCD,* HlyB→css← | this work |
| Chcfa1-R1 | CHcfaI-1 *hlyB::rns-1*/*cfaABCE* | this work |
| BB01 | CHcfaI-1 *hlyB::rns-1*/*cfaABCE* | this work |
| CHCS3-R2 | CHCS3-2 *hlyB::rns*/*cstABC* | this work |
| BB06 | CHCS3-R2 *hlyA::cfaABCE, hlyA*→cfa→ | this work |
| BB07 | CHCS3-R2 *hlyA::cfaABCE, hlyA*→cfa← | this work |
| BB06 (pSSVI215-CS6) | | this work |
| BB07 (pSSVI215-CS6) | | this work |

| plasmids | | |
|---|---|---|
| pK18 | Km^{r}, 2.4 Kb, high copy number cloning plasmid | R.Pridmore |
| pCL1920 | Spc^{r}, 4.7 Kb low-copy number plasmid | C.Lerner |
| pMAKSACA | Cm^{r}, low copy number, sacB, rep101ts, suicide integration vector | Favre and Viret, 2000 |
| pMAKSACB | Cm^{r}, low copy number, sacB, rep101ts, suicide integration vector | Favre and Viret, 2000 |
| pSSVI215 | Km^{r}, low copy number, cos | Favre et al, 1996 |
| pM295 | source of *cssABCD* (CS6) locus | M.K. Wolf |
| pGB1 | source of *cstABC* (CS3) locus | D.A. Scott |
| pM392 | plasmid carrying the *csfR* regulator gene | M.K.Wolf |
| pM315 | source of the *csfR* regulator gene | M.K. Wolf |
| pEU2040 | source of the *rns* regulator gene | J.R.Scott |
| pJMK10 | Source of *hlyAB* locus | Ketley et al, 1993 |
| pCLRNS | pCL1920 with the 1.3 Kb *Hind*III*-Eco*RI *rns* fragment from pEU2040 | this work |
| pK18cfaI-a | *cfaI* locus cloned as a Psp1406I fragment in pK18 restricted by Smal | this work |
| pSSVI215-cfaI | *cfaI* locus cloned as a 7.3 Kb *Sac*I fragment in pSSVI215 cut by *Sac*I | this work |
| pSSVI215-CS3 | CS3 locus cloned as a 4746 bp *Hin*dIII fragment in *Hin*dIII-cut pSSVI215 | this work |
| pSSVI215-CS6 | CS6 locus cloned as a 5218 bp *Sac*I fragment in *Sac*I-cut pSSVI215 | this work |
| pMAKSACAhly | *hlyAB* locus as a 3559 bp *Pin*AI*-Pst*I fragment from pJMK10 cloned in *Xmal-Pst*I-cut pMAKSACA | this work |
| pMAKcfaI-Km1 | 8.6 Kb cfaI-Km^{r} *Swa*I fragment from pSSVI215-cfaI cloned in *Nru*I-cut pMAKSACAhly | this work |
| pMAKcfaI-Km2 | as pMAKcfaI-Km1 but cfaI-Km^{r} fragment in opposite orientation | this work |
| pMAKcfaI-1 | pMAKcfaI-Km1 restricted by I-Scel and self ligated to remove the Km^{r} gene. Integration plasmid for the CFA/I locus. Transcription of CFA/I locus in the same direction as the hlyB gene | this work |
| pMAKcfa-2 | pMAKcfaI-Km2 restricted by I-Scel and self ligated to remove the Km^{r} gene. Integration plasmid for the CFA/I locus. Transcription direction of CS6 locus opposite to that of the *hlyB* gene | this work |
| pSSVI215-cfaI/S | *cfaI* locus cloned as a 5.6 Kb *Eco*RV-*Sac*I fragment in pSSVI215 cut by *Bam*HI/Klenow and *Sac*I | |
| pMAKcfaISKm-4 | 6.9 Kb cfaI-Km^{r} *Not*II fragment from pSSVI215-cfaI cloned in *Nru*I-cut pMAKSACAhly | |
| pMAKcfaIS-4 | pMAKcfaISKm-4 restricted by I-*Sce*I and self ligated to remove the Km^{r} gene. Integration plasmid for the CFA/I locus. Transcription of CFA/I locus in the same direction as the *hlyB* gene | |
| pMAKCS3-Km2 | 6 Kb Swal CS3-Km^{r} fragment from pSSVI215-CS3 cloned in *Nru*I-cut pMAKSACAhlyA | this work |
| pMAKCS3-2 | pMAKCS3-Km2 restricted by I-Scel and self ligated to remove the Km^{r} gene. Integration plasmid for the CS3 locus | this work |
| pMAKCS6-Km1 | 6 Kb Swal CS3-Km^{r} fragment from pSSVI215-CS6 cloned in *Nru*I-cut pMAKSACAhlyA | this work |
| pMAKCS6-Km2 | as pMAKCS6-Km1 but CS6-Km^{r} fragment in opposite orientation | this work |
| pMAKCS6-1 | pMAKCS6-Km1 restricted by I-Scel and self ligated to remove the Km^{r} gene. Integration plasmid for the CS6 locus. Transcription of CS6 locus in the same direction as the *hlyB* gene | this work |
| pMAKCS6-2 | pMAKCS6-Km1 restricted by I-Scel and self ligated to remove the Km^{r} gene. Integration plasmid for the CS6 locus. Transcription direction of CS6 locus opposite to that of the *hlyB* gene | this work |
| pMAKSACBhlyAR | 1653 bp *Bam*HI - *Nsi*I fragment from pJMK10 encompassing the 3'-end of the *hlyA* gene and the 5'-end of the *hlyB* gene cloned in *Bam*HI-*Nsi*I restricted pMAKSACB. | this work |
| pMAKcsfR-1 | a 1121 bp *Ssp*I fragment from pM392 into pMAKSACBhlyAR restricted by *Eco*RV. Transcription direction of *csfR* locus opposite to that of the *hlyB* gene | this work |
| pMAKcsfR-2 | *csfR* in opposite orientation with respect to that in pMAKcsfR-1 | this work |
| pMAKrns-1 | 1.2 Kb *Eco*RI-*Hind*III fragment from p into pMAKSACBhlyAR restricted by *Eco*RV | this work |
| pMAKrns-2 | rns in opposite orientation with respect to that in pMAKrns-1 | this work |
| pSSVI207-2 | Cosmid containing the *hly::mer* region of CVD 103-HgR | this work |
| pMAKSACBhlyAL | 2.23 Kb *Sca*I*-Bgl*II fragment from pSSVI207-2 cloned into vector pMAKSACB cut with *Bam*HI and *Sma*I*.* | this work |
| pMAKhlyAcfaIS-3 | klenowed *Not*I fragment from pSSVI215-cfaI/S subcloned into *Nru*I site of pMAKSACBhlyAL. CFA/I operon transcribed in the same direction as *hlyA* | this work |
| pMAKhlyAcfaIS-4 | Same as pMAKhlyAcfaIS-3 but insert in reverse orientation | this work |

All *E. coli* K12 strains used for cloning, as well as enterotoxigenic *E*. *coli* were grown in LB (Luria broth) medium (Sambrook, Fritsch and Maniatis, 1989). For solid medium, 2% agar was added. Antibiotics were used at the following concentrations: chloramphenicol 17 µg/ml, kanamycin 50 µg/ml. For all strain construction purposes *V. cholerae* was grown on LBSOY consisting of 10g/L Soytone tryptone (Difco), 5 g/L Yeast extracts (Difco), and 10g/L NaCl, pH 7.3, or LBHySoy consisting of 10 g/L HySoy (Quest International, Bussum, Netherlands), 5 g/L Yeast extracts (Difco), and 10g/L NaCl, pH 7.2.

Pili expression was obtained by growing the recombinant *E*. *coli* or *V. cholerae* strains in CF medium consisting of 10.0 g/L Casamino acids, 1.5 g/L Yeast extracts, 50 mg/L MgSO₄ x 7H₂O, and 5 mg/L MnCl₂ x 4H₂O, or 2xCF medium consisting of 20.0 g/L Casamino acids, 10.0 g/L Yeast extracts, 100 mg/L MgSO₄ x 7H₂O, and 10 mg/L MnCl₂ x 4H₂O. The latter medium allowed a similar or better expression of pili than CF, while allowing a higher cell density to be reached. The pH of CF was adjusted to either 6.5 or 7.2 by NaOH. 2xCF medium was used at a pH of 7.3. In some experiments 5g/L lactose was added to the medium (2xCFL medium). In cell adherence experiments, the bacteria were assayed on BHI plates (DIFCO).

Caco-2 cells for the adherence assay were seeded at 10⁴ cells/well and grown at 37°C under 5% CO₂ in MEM medium until almost confluent.

### 2. Cloning of pili loci into integration vectors

### 2.1. Preparation of the integration vectors

The integration of different DNA loci in the same strain has to occur at a specific location for each DNA locus. Therefore a total of two different integration vectors will be described in this Example, reflecting the integration of two different pili operons in the same strain.

### 2.1.1. Vector for the integration of the first pili operon

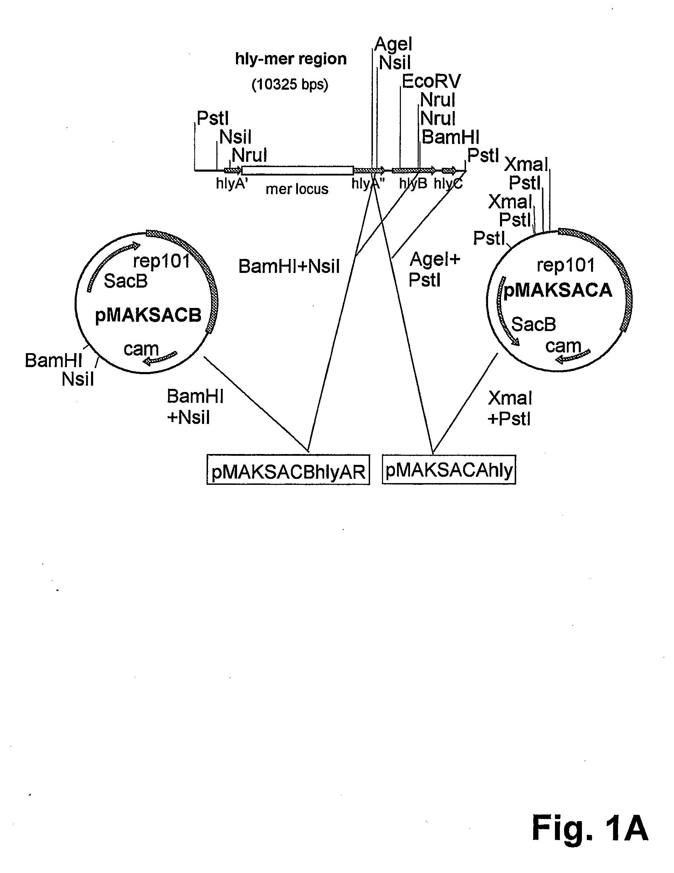

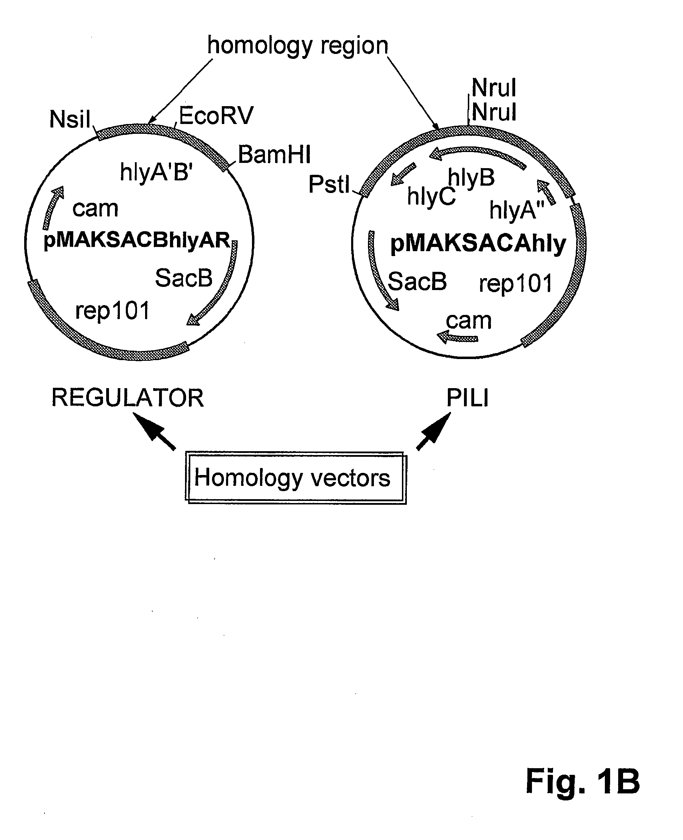

The integration vector was based on the suicide integration plasmid pMAKSACA (Favre and Viret, 2000) which was restricted with *Xma*I and *Pst*I*.* The homology region derived from the plasmid pJMK10 (Ketley, et al, 1993), corresponding to a 3559 bp *Pin*AI*-Pst*I fragment encompassing the 3'-end of the *hlyA* gene, the entire *hlyB* gene and the 5'-end of the IipA (hlyC) gene, was cloned in the restricted vector to produce the vector pMAKSACAhly. pMAKSACAhly contains two *Nru*I sites which are located 8 bp apart in the middle of the insert and within the *hlyB* gene. These *Nru*I sites can be used for cloning of foreign genes to produce integration plasmids (Figure 1).

### 2.1.2. Vector for the integration of the second pili operon

Strain CHCS3-R2 was taken as the host strain for the integration of the CFA/I operon. The integration vector pMAKSACBhlyAL was constructed by cloning a 2.23 Kb *Sca*I*-Bgl*II fragment from pSSVI207-2 into vector pMAKSACB cut with *Bam*HI and *Sma*I*.* The *Sca*I*-Bgl*II fragment encompasses 0.93 Kb of sequence upstream of the *hlyA* gene, the 5' part of the *hlyA* gene, the *mer*R gene and the 5' part of the mer operon in CVD 103-HgR. In this context integration of a foreign gene can be effected at the single *Nru*I site located within *hlyA,* 203 bp downstream from the start codon.

### 2.2. Subcloning of the pili loci

### 2.2.1. CS3

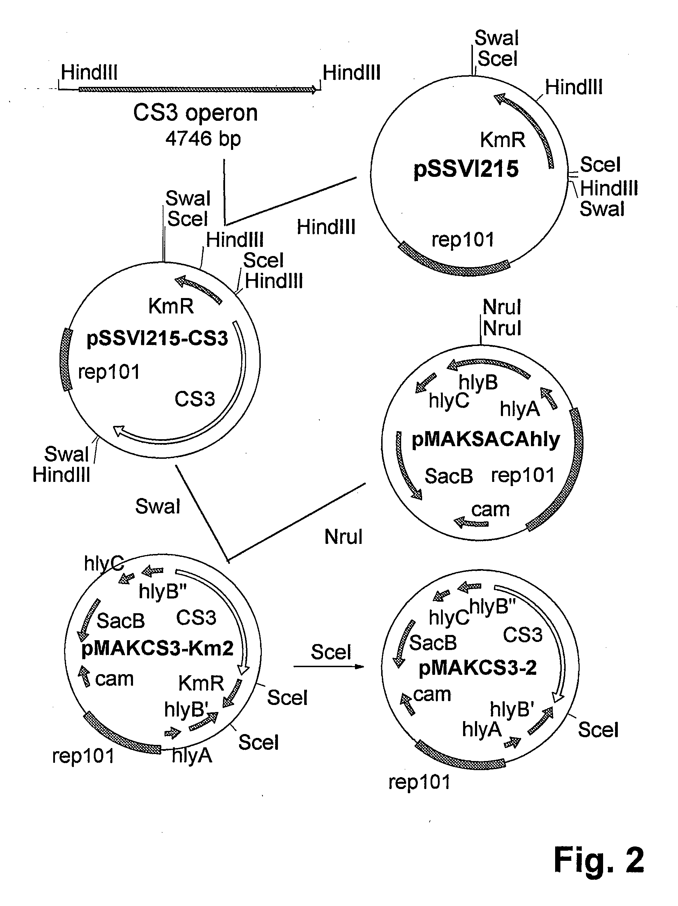

The CS3 operon was excised from plasmid pGB1 as a 4.7 Kb *Hind*III fragment. This fragment was first cloned in the low-copy number vector pSSVI215 restricted by *Hin*dIII. The resulting plasmid was called pSSVI215-CS3 and contains the CS3 operon in tandem with a kanamycin-resistance gene (KmR) which serves as a positive marker for the subsequent subcloning. The 6 Kb CS3 operon-KmR gene fragment was further isolated by restricting pSSVI215-CS3 with Swal and subcloned in pMAKSACAhly restricted by *Nru*I*.* This produced the plasmid pMAKCS3-Km2, whereby the homology region is split between a left-hand segment of 1770 bp and a right-hand segment of 1781 bp. In order to remove the kanamycin-resistance gene, pMAKCS3-Km2 was excised by *Sce*I and the large fragment was self-ligated. This resulted in the final 14.7 Kb integration plasmid called pMAKCS3-2 (Figure 2).

### 2.2.2. CS6

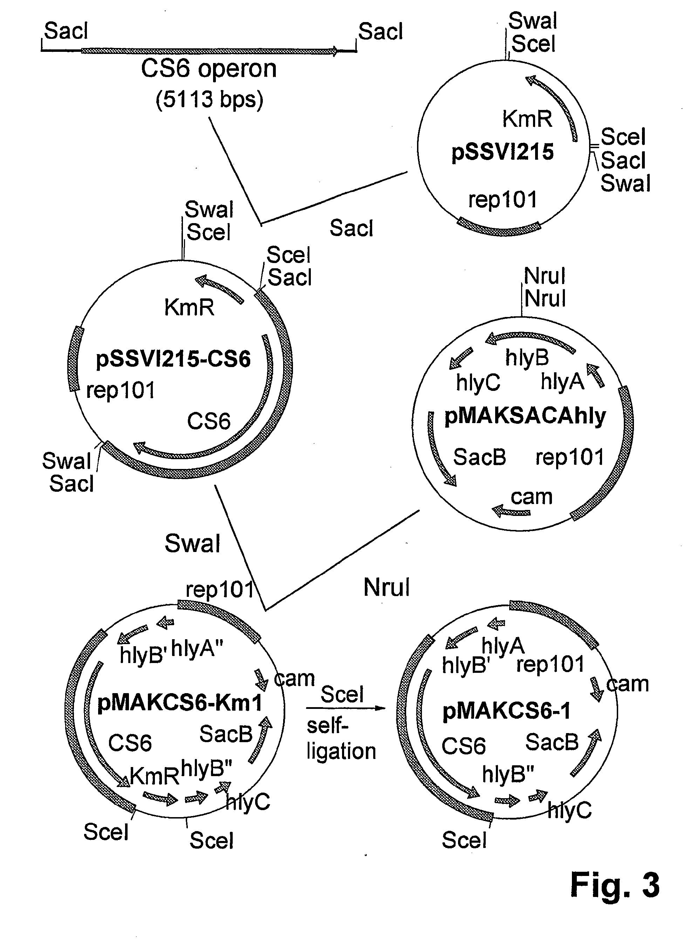

The CS6 operon was excised from plasmid pM295 as a 4.5 Kb *Sac*I fragment. This fragment was first cloned in the low-copy number vector pSSVI215 restricted by *Sac*I. The resulting plasmid was called pSSVI215-CS6 and contains the CS6 operon in tandem with a kanamycin-resistance gene (KmR) which serves as a positive marker for the subsequent subcloning. The ca. 6 Kb CS6 operon-KmR gene fragment was further isolated from pSSVI215-CS6 by restriction with Swal and subcloned in pMAKSACAhly restricted by *Nru*I*.* This produced the plasmid pMAKCS6-Km-1 and pMAKCS6-Km-2, whereby the former plasmid contains the *cssA, B**,** C**,*** and *D* genes of the CS6 operon in the same orientation as the *hlyB* gene whereas the latter plasmid has the operon in opposite orientation to *hlyB.* The interruption of the *hlyB* gene by the CS6 locus produces a left-hand homologous segment of 1770 bp and a right-hand homologous segment of 1781 bp. In order to remove the kanamycin-resistance gene, pMAKCS6-Km-1 and pMAKCS6-Km-2, were excised by Scel and the large fragments were self-ligated. This resulted in the final 14.5 Kb integration plasmids called pMAKCS6-1 and pMAKCS6-2. Figure 3 shows the construction of pMAKCS6-1.

### 2.2.3. CFA/I

### 2.2.3.1. Long CFA/I insert

The entire CFA/I operon was excised from plasmid pK18cfaI-a as a 7.3 Kb *Sac*I fragment. This fragment was first cloned in the low-copy number vector pSSVI215 restricted by *SacI.* The resulting plasmid was called pSSVI215-cfaI and contains the CFA/I operon in tandem with a kanamycin-resistance gene (KmR) which serves as a positive marker for the subsequent subcloning. The ca. 8.6 Kb CFA/I operon-KmR gene fragment was further isolated from pSSVI215-cfaI by restriction with *SwaI* and subcloned in pMAKSACAhly restricted by *NruI.* This produced the plasmids pMAKcfaIKm-1 and pMAKcfaIKm-2, whereby the former plasmid contains the genes of the CFA/I operon in the same orientation as the *hlyB* gene whereas the latter plasmid has the CFA/I operon in opposite orientation to *hlyB.* The interruption of the *hlyB* gene by the CFA/I locus produces a left-hand homologous segment of 1770 bp and a right-hand homologous segment of 1781 bp. In order to remove the kanamycin-resistance gene, pMAKcfaIKm-1 and pMAKcfaIKm-2 were excised by *SceI* and the large fragments were self-ligated. This resulted in the final 17.2 Kb integration plasmids called pMAKcfaI-1 and pMAKcfaI-2, respectively.

### 2.2.3.2. Short CFA/I insert

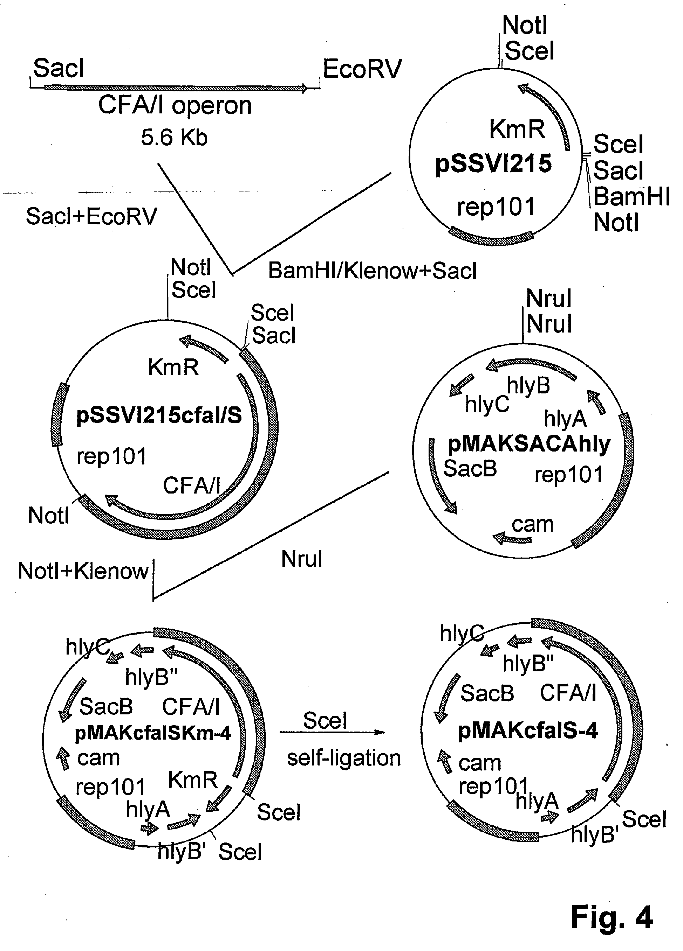

Due to genotypic stability problems with strain CHcfaI-RI, a second CFA/I-producing strain was constructed from which most of the Δ*cfa*D gene was eliminated. Possibly, the presence of the RNS regulator in the vicinity of the nearly homologous defective regulator (Δ*cfa*D) at the 3'-end of the CFA/I operon has allowed recombination events to occur with resulting unstability. The CFA/I locus was cloned from plasmid pK18cfaI-a as a 5563 bp *Eco*RV-SacI fragment lacking most of the Δ*cfaD* gene and cloned into plasmid pSSVI215 to produce pSSVI215-cfaI/S. The CFA/I locus was further subcloned together with the adjacent kanamycin-resistant gene in the middle of the *hlyB* gene in the integration plasmid pMAKSACAhly cut by *Nru*I to produce pMAKefaIS-Km-4. The kanamycin-resistant gene was removed from pMAKcfaIS-Km-4 by SceI restriction and self-ligation. The resultant plasmid, called pMAKcfaIS-4, was introduced by electroporation in CVD 103-HgR. The CFA/I locus was then chromosomally integrated within the *hlyB* gene of the host strain. The resulting strain was called CHcfaI-4. In this strain the CFA/I genes are transcribed in the same orientation as the *hlyB* gene. Figure 4 shows the construction of the plasmids.

### 3. Construction of an integration vector for integration of the regulator genes

The integration vector was based on the suicide integration plasmid pMAKSACB (Favre and Viret, 2000) which was restricted with *Bam*HI and Nsil, creating a 6724 bp vector. The homology region corresponded to a 1653 bp *Bam*HI *- Nsi*I fragment derived from pJMK10 (Ketley et al, 1993) encompassing the 3'-end of the *hlyA* gene and the 5'-end of the *hlyB* gene. The latter fragment was cloned into pMAKSACB to create pMAKSACBhlyAR. The latter vector contains a unique *Eco*RV cloning site within the homology region, providing 885 and 772 bp of homology arms, and located 245 bp downstream of the ATG start codon of the *hlyB* gene (Figure 1).

### 4. Cloning of regulators Into integration vectors

### 4.2. Rns

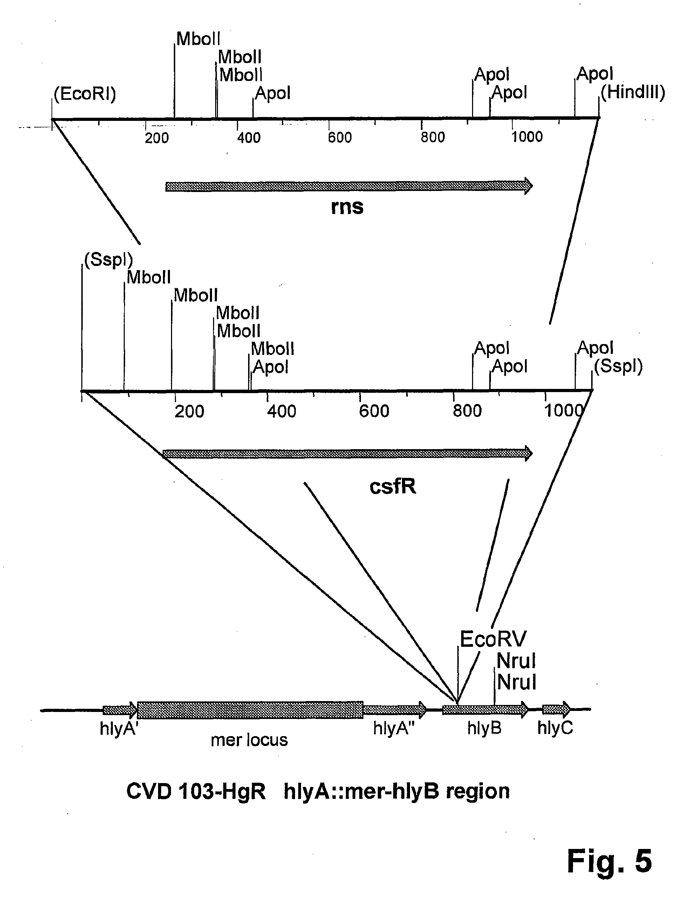

The ms regulator gene was cloned as a ca., 1.2 Kb Klenowed *EcoR*I-*Hin*dIII fragment into pMAKSACBhlyAR restricted by *Eco*RV. Two orientations of the gene within the vector were found by restriction analysis of the plasmids with *Alw*NI and called pMAKms-1 and pMAKms-2. In plasmid pMAKrns-1, ms is transcribed in the reverse orientation with respect to *hlyB* gene transcription, whereas in pMAKrns-2, *rns* is transcribed in the same orientation as *hlyB* (Figure 5).

### 5. Integration of pill and regulator loci into the chromosome of V. cholerae

### 5.1. Pili loci

### 5.1.1. Construction of monovalent strains

In a first phase, the plasmids pMAKCS3-2, pMAKCS6-1, pMAKCS6-2, pMAKcfaI-1, pMAKcfaI-2, and pMAKcfaS-4 were transferred in the *V. cholerae* live attenuated recipient vaccine strain CVD 103-HgR by either transformation or conjugation via E. coli K12 S17.1 (Simon et al, 1983). In all subsequent steps, care was taken to grow cells on either the bovine-free medium called LBSOY consisting of 10g Soytone tryptone (Difco), 5 g Yeast extracts (Difco), and 10g NaCl or LBHySoy consisting of 10g HySoy (Quest), 5 g Yeast extracts (Difco), and 10g NaCl. For solid LBSOY or LBHySoy, 2% agar was added. Where needed, 17 µg/ml chloramphenicol was added to the plates (LBSOY Cm or LBHySoy Cm). Growth from a LBSOY Cm plate with CVD 103-HgR (pMAKCS6-1) and (pMAKCS6-2) was inoculated in 1 ml PBS and homogenized. Growth from CVD 103-HgR (CHCS3-2), CVD 103-HgR (pMAKcfaI-1), CVD 103-HgR (pMAKcfaI-2), and CVD 103-HgR (pMAKcfaIS-4) was inoculated in 2 mls 0.9% NaCl and homogenized. 100 µl of 1x, 10x, and 100x dilutions of these cell suspensions were plated out onto LBSOY Cm or LBHySoy Cm plates and incubated at 42°C for 16 hours. Individual colonies were then gathered and streaked out onto a 42°C pre-warmed LBSOY Cm or LBHySoy Cm plate and the plate was incubated at 42°C. Several of the resulting colonies were inoculated in 2 ml of liquid LBSOYS-N or LBHySoy-N and incubated at 30°C during several hours. LBSOYS-N or LBHySoy-N is LBSOY or LBHySoy without NaCl and with 5% sucrose added. This medium allows the selection of integrants which have lost the vector sequences. The OD₆₀₀ of the cultures was measured and the cultures were serially diluted in order to have 100 to 10'000 CFU/plate. 100 µl dilutions were plated out onto LBSOYS-N or LBHySoyS-N plates, which were incubated at 30°C for 16 to 24 hours. Next, the plates containing 100 to 500 colonies were successively replica-plated onto LBSOYS-N Cm or LBHySoyS-N Cm and LBSOYS-N or LBHySoyS-N. After overnight growth at 30°C, the colonies on both media were compared and 50 Cm-sensitive colonies were short-streaked onto new LBSOYS-N or LBHySoyS-N plates and grown at 30°C over night (O/N). These plates were subsequently blotted onto nylon filter and the presence of the CFA/I, CS3, and CS6 loci was tested by colony hybridization using specific DIG-labeled probes. A number of strains were found which have lost the vector sequences (Cm-sensitive) while conserving the pili loci. The new strains issued from pMAKCS3-2, pMAKCS6-1, pMAKCS6-2, pMAKcfaI-1, pMAKcfaI-2, and pMAKcfaIS-4 were named CHCS3-2, CHCS6-1, CHCS6-2, CHcfaI-1, CHcfal-2, and CHcfal-4, respectively.

### 5.1.2. Construction of bivalent strains

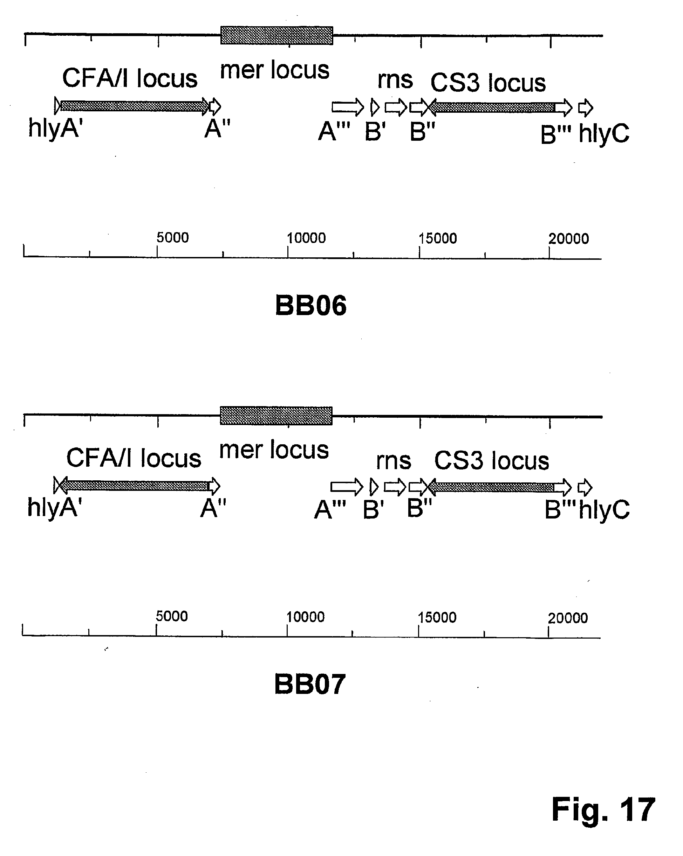

Both pMAKhlyAcfaIS-3 and pMAKhlyAcfaIS-4 plasmids were electroporated into CHCS3-R2. Subsequently, integration procedure was followed as above for monovalent operon integration (see Example 5.1). Colonies of CHCS3-R2 having an integrated copy of the CFA/I operon were identified for both the pMAKhlyAcfaIS-3 and pMAKhlyAcfaIS-4 plasmids and called BB06 and BB07, respectively (Figure 17).

### 5.2. Regulators

Plasmids pMAKcsfR-1, pMAKcsfR-2, pMAKrns-1, and pMAKrns-2 were electroporated in CHcfaI-1, CHcfaI-2, CHcfal-4 and CHCS3 *V. cholerae* strains containing integrated pili loci. Each strain was grown onto either LBSOY or LBHySoy medium from glycerol stock cultures. The electroporation cultures were plated out onto LBSOY Cm or LBHySoy Cm medium and grown at 30°C. The integration procedure was then followed exactly as for the pili loci. Following integration, various integrants were screened for production of pilin. Three types of strains could thus be isolated, corresponding to CHcfaI-1 having integrated the rns gene from plasmid pMAKrns-1, to CHcfal-4 having integrated the *rns* gene from pMAKrns-1 and to CHCS3-2 having integrated the rns gene from plasmid pMAKrns-2 on the other hand. The strains were called CHcfaI-R1, BB01 and CHCS3-R2, respectively.

Some combinations of pili recombinant strains and regulator genes did not allow any pilin production, demonstrating that various factors may affect the expression of pilin from a combination of pili operon and regulator gene. Thus, the choice of such a combination is not a trivial one.

### 6. Western Blot assays of pili expression with or without regulator after growth on solid CF agar

The expression of pilin, the structural protein of the pili was studied by Western blotting. The strains to analyze were grown as lawns overnight on CF agar, pH 6.5 at the indicated temperature. Part of the lawn of bacteria was resuspended in 2 mls of 0.9% NaCl, the OD₆₀₀ was measured and the cell density was adjusted to an OD₆₀₀ of 3.0. One volume of the cell suspension was then mixed with 1 volume of SDS-PAGE sample buffer. 15 µl were subsequently run on a Criterion™ Precast Gel 4-20% (BioRad Laboratories, Glattbrugg, Switzerland). Following blotting against a nitrocellulose membrane, the membrane was blocked in 10% powdered milk (Rapilait, Migros) for 1 h at 37°C and incubated in the presence of a 10'000 dilution of pili-specific rabbit polyclonal antibodies. After three consecutive washes in PBS-T the membrane was incubated with a 10'000-fold dilution of a HP-conjugated goat anti-rabbit IgG (H + L). The membrane was again washed and the bands were detected by chemoluminescence after a 5 min contact with 1 ml ChemiGlow™ West Luminol / Enhancer Solution (Alphalnnotech, San Leandro, USA). The bands were detected using a video camera device (Chemilmager, Alphalnnotech, San Leandro,USA).

### CS3

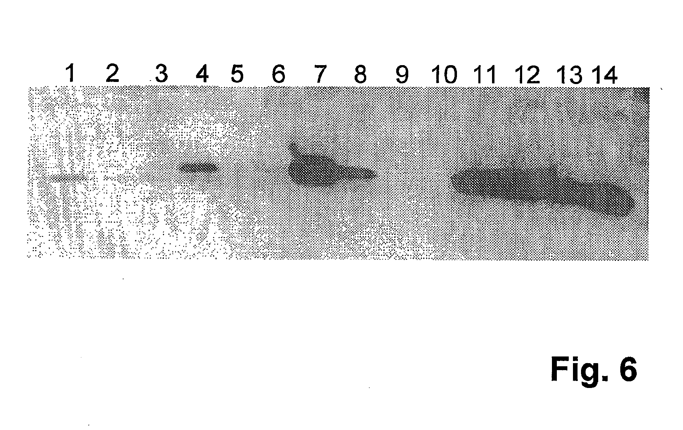

As shown in Figure 6, the production of pilin from both the *E*. *coli* K12 and *V. cholerae* CVD 103-HgR hosts appears to be dependent on the plasmid copy number. Thus in both cases expression is higher when the CS3 locus is expressed from plasmid pGB1 which is present at about 25 copies/cell (lanes 4 and 8) than from pSSVI215-CS3 which is present at about 3-5 copies/cell (lanes 3 and 6). Surprisingly, however, CS3 expression is vastly increased by co-expression of the *csfR* regulator, here carried on plasmid pM392. The action of the regulator is all the more spectacular that CS3 expression appears to be just as strong from the CS3 integrated locus as it is from the low-copy number clone pSSVI215-CS3 (compare lane 7 with lane 12 and 14). In contrast, the expression of the integrated locus in the absence of the regulator is extremely poor (lanes 9 and 10). Another surprising finding was that CS3 expression in the presence of the regulator was similar when the cells were grown at 30°C or 37°C. This is in contrast to the current understanding that in the wild type strain, pili expression is markedly better at 37°C than at 30°C.

### CS6

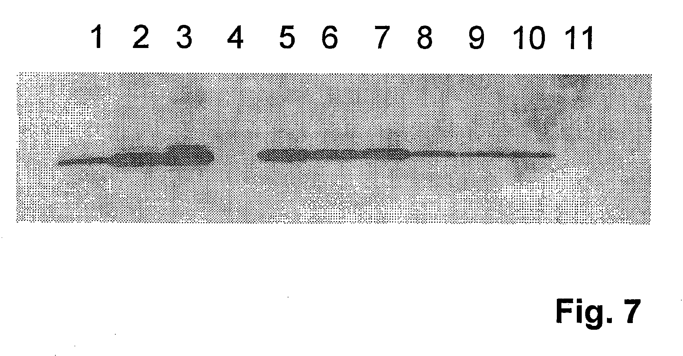

In contrast to CS3, CS6 pilin appears to be synthesized in the absence of any regulator (Figure 7, lanes 5 and 7). As a corollary, the expression of CS6 is dependent on plasmid copy-number. Thus, the highest amount of CS6 pilin stems from the high-copy-number plasmid pM295, followed by pSSVI215-CS6, and the integrated strain CHCS6-1 and CHCS6-2. However, it is noteworthy that even in the latter case, as much pilin is made as in the ETEC wild-type strain B7A. This underscores the fact that expression in CVD 103-HgR is quite efficient, even from a single copy locus. As for CS3, it appears that, in CVD 103-HgR the expression of CS6 is as good at 30°C than at 37°C.

### CFA/I

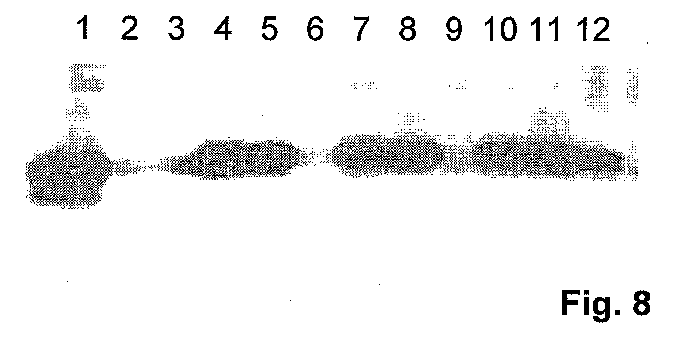

The expression profile of CFA/I pilin resembles that of CS3. Figure 8 shows that, surprisingly, expression is highly dependent on the *csfR* regulator irrespective of whether the CFA/I locus is present on a plasmid (lanes 3 and 5) or chromosomally integrated (strains CHcfaI-1 and CHcfal-2, compare lanes 6 and 8, as well as lanes 9 and 11). This appears to be in contrast with expression of CFA/I in foreign host which did not require a regulator (Wu et al, 1995). As in CS3, expression of CFA/I in the presence of the regulator is independent of the number of copies of the CFA/I locus since there is no difference in the amount of pilin made by the integrated strain or by the plasmid-encoded locus (compare lane 5 with lanes 8 or 11). Without a regulator, expression depends on the plasmid copy-number. Expression from the high-copy number pK18cfaI-1 (lane 12) is higher than that from the low-copy number plasmid pSSVI215-cfaI (lane 3). In contrast to the findings of Wu et al., expression of the locus in the absence of the regulator is poor (lanes 3, 6, and 9). A further surprising finding was that CFA/I expression in the presence of the regulator was similar when the cells were grown at 30°C or 37°C.

### 7. Western Blot assays of pili expression using either the CsfR or the Rns regulator

In a further aspect, it was checked whether the use of any regulator was suitable to express CS3 and CFA/I pili. Accordingly, the *rns* regulator gene carried on plasmid pEU2040 (high copy number plasmid) or on pCLrns (low-copy number plasmid) was electroporated in strains CHcfaI-1, CHcfal-2, and CHCS3-2, and the expression of CFA/I and CS3, respectively, was analyzed by Western blot.

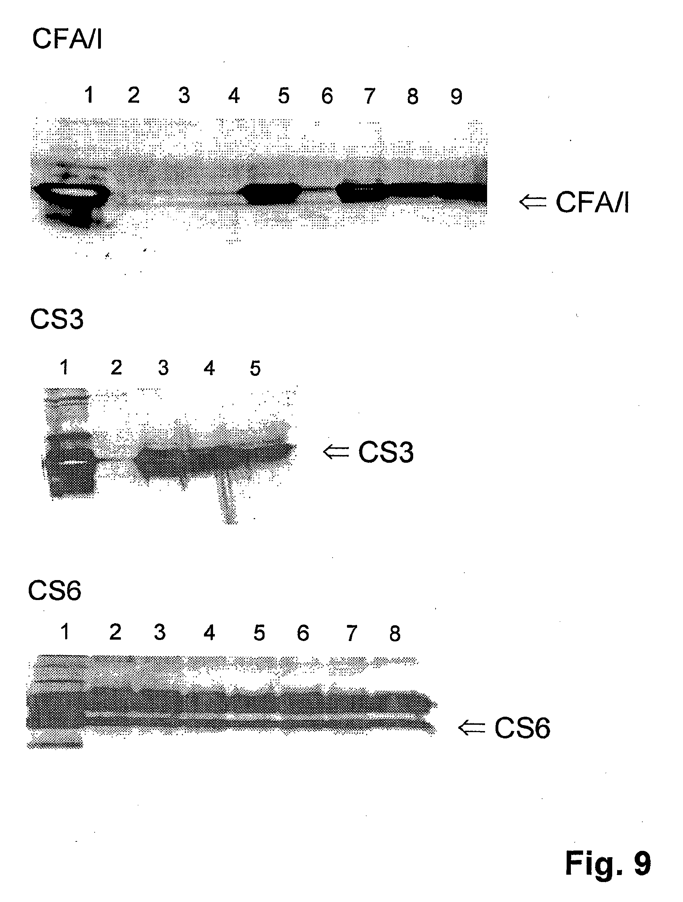

As the results depicted in Figure 9 show, the expression from the Rns or CsfR regulators is very similar in magnitude. The only visible difference occurs in CHcfaI-1. In this strain, the CsfR regulator allows strong expression of the CFA/I pilin whereas the Rns regulator does not. This shows that, in contrast to the current knowledge, the CsfR and Rns regulators are not completely interchangeable.

### 8. Western Blot assays of pili expression after growth in liquid CF medium

Current knowledge holds that the ETEC pili (and, presumably pilins) are best expressed on solid CF plates. It was therefore checked if this holds true in the case of *V. cholerae-*expressed pilin. In addition, the effect on pilin expression of CF medium at either pH 6.5 or pH 7.3 was compared. Overnight LBHySoy cultures of the strains to test were either spread as lawns of cells on a CF or chloramphenicol-containing CF (CFCm) plate, pH 6.5, or diluted in 10 mls of CF or CFCm medium, pH 6.5 or 7.3. Liquid and solid cultures were grown at 37°C. From the CF/CFCm plates, cell suspensions in 0.9% NaCl were made to an OD₆₀₀ of 3.0. These suspensions served as reference for production of pilin from solid CF. The overnight CF/CFCm cultures were diluted to an OD₆₀₀ of 0.1 in 30 ml of fresh CF/CFCm medium at pH 6.5 or 7.3 and the cultures were agitated at 37°C. At OD₆₀₀ of 0.5, 1.5, as well as after overnight growth, samples of the cultures were withdrawn, pelleted and resuspended to an OD₆₀₀ of 3.0 in 0.9% NaCl. Finally, 100 µl of the cell suspensions at OD₆₀₀=3.0 were mixed with 100 µl of sample buffer and 15 µl of each sample was run on a 4-20% gradient SDS-PAGE gel. After the run, the gel was blotted onto nitrocellulose membrane and the pilin proteins were detected by means of specific polyclonal antibodies.

The results are presented in Figure 10. Surprisingly, pilin from any of the pili types, i.e., CFA/I, CS3, or CS6 was very well expressed in liquid CF. There is no significant difference in the amount of pilin made from bacteria grown on plates or in liquid medium. Whereas CS6 pilin is made in equivalent amounts at all growth phases, CS3 and CFA/I pilin seem to be made in larger amount in exponential phase than in stationary phase. The difference is however rather small. In addition, there is no difference between growth in CF medium pH 6.5 or 7.3. In conclusion, pilin is very well synthesized by *V. cholerae* strains carrying pili loci and regulators grown in liquid cultures.

### 9. Expression of pilin from recombinant strains with an integrated regulator gene.

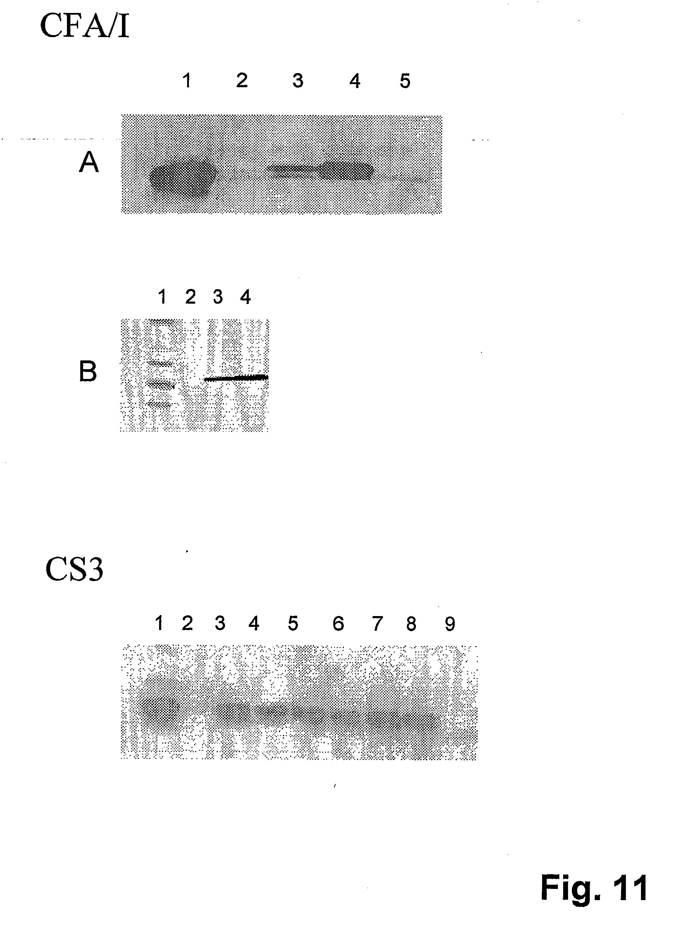

The amount of pilin synthesized by strains containing the integrated *rns* regulator gene was tested by Western blotting. Figure 11, panel CFA/I, A indicates excellent synthesis of CFA/I pilin by CHcfa1-R1 strain (lane 4) even compared to the CVD 103-HgR carrying a plasmid-borne *rns* gene (compare lanes 3 and 4). Similarly, panel B shows that CFA/I pilin synthesis from strain BB01 and the wild type ETEC strain CD79a are comparable. Therefore, the integration of the *rns* gene leads to a very good expression of the CFA/I pilin. Likewise, panel CS3 shows that the expression of the CS3 pilin is just as strong from the CHCS3-R2 strains which possess the integrated *rns* gene (lanes 5-8), as it is from strains carrying the plasmid-borne *rns* gene (lanes 3-4).

### 10. Co-expression of two pilis in the same recipient strain

It was investigated the possibility to co-express two different pilis in a single strain. CHCS3-R2 was chosen as the recipient strain because of its excellent surface expression of the CS3 pili. In preliminary experiments, the plasmids pSSVI215-cfaI or pSSVI215-CS6 were electroporated into CHCS3-R2. The expression of both the CS3 and CFA/I pilis in strain CHCS3-R2 (pSSVI215-cfaI), as well as the expression of the CS3 and CS6 pilis in the strain CHCS3-R2 (pSSVI215-CS6) were studied by Western blotting, immunospots and immunofluorescence. The expression of both the CFA/I and CS3 pili in the double-integrant strains BB06 and BB07 was studied by Western blot and pili ELISA (for the results of the latter see table 8).

### 10.1. Western blot

A Western blot was carried out as described above (see example 6). The results are shown in table 4.

### 10.2. Immunospots immunofluorescence

To determine the immunofluorescence, the method of Lang et al, 1990 was followed. Briefly, the strains indicated in table 4 were grown onto CF plates at 37°C. A cell suspension was then made in 0.9% NaCl at an OD₆₀₀ of 1.0-2.0. A loopful of the suspension was smeared on a glass slide and air-dried. The slides were then dipped in cold acetone (-20°C) for 10 min at 4°C and air-dried. The glass slides were washed 3 x in PBS, air dried, and 100 µl of a 50 times dilution of each of the specific antisera were applied to the corresponding dried cells and incubated at room temperature for 15 min. The slides were again washed 3x in PBS, air-dried and 100 µl of a 1:100 dilution of a FITC-labeled anti-rabbit IgG antibody was applied to the cells at room temperature for 15 min. The slides were again washed 3x by PBS and air-dried. Finally, 5 µl H₂O and a drop of MOVIOL was added to each bacterial spot and the preparation was covered with a cover slip. The results were recorded on a Nikon Eclipse E800 fluorescence microscope.

For detection of immunospots, the strains indicated in table 4 were grown in CF medium, resuspended in 0.9% NaCl to an OD₆₀₀ of 3.0 and diluted to an OD₆₀₀ of 0.3. 5 µl of the undiluted and diluted cell suspensions were spotted on a nitrocellulose filter. The filter was blocked with 10% horse serum. Then the filter was incubated for 1 h with a 1:10'000 dilution of the specific polyclonal antibody at room temperature. After extensive washing the filter was further incubated for 30 min at room temperature with a 1:10'000 dilution of a horseradish peroxidase-conjugated anti-rabbit IgG antibody. After washing, the filter was either incubated with a chemolumiescent substrate or with a colorimetric substrate. In the former case, the development of chemoluminescence is detected with a video camera device (Chemilmager, Alphalnnotech, San Leandro CA, USA) or by colorimetric development of the signal.

**Table 4: Co-expression of pilis with CHCS3-R2 as the recipient strain**

| Primary antibody | Strain | Western blot^{a} | Immunospots^{b} | Immunofluorescence^{c} |
|---|---|---|---|---|
| | H10407 | - | + | + |
| | DS7-3 | + | ++ | +++ |
| | B7A | - | - | - |
| CS3 | CHCS3-R2 | +++ | ++ | ++ |
| | CHcfa1-R1 | - | - | - |
| | CHCS6-1 | - | - | - |
| | CHCS3-R2 (pSSVI215-cfaI) | +++ | ++ | ++ |
| | CHCS3-R2 (pSSVI215-CS6) | +++ | ++ | ++ |
| | BB06 | +++ | ND^{d} | ND |
| | BB07 | +++ | ND | ND |
| | CVD 103-HgR | - | - | - |
| | | | | |
| | H10407 | ++ | ++ | +++ |
| CFA/I | DS7-3 | - | - | - |
| | CHCS3-R2 | - | - | - |
| | CHcfa1-R1 | + | ++ | ++ |
| | CHCS3-R2 (pSSVI215-cfaI) | ++ | ++ | ++ |
| | BB06 | ++ | ND | ND |
| | BB07 | ++ | ND | ND |
| | CVD 103-HgR | - | - | - |
| | | | | |
| | B7A | ++ | + | +++ |
| | DS7-3 | - | - | - |
| CS6 | CHCS3-R2 | - | + | - |
| | CHCS6-1 | + | + | - |
| | CHCS3-R2 (pSSVI215-CS6) | +++ | ++ | + |
| | CVD 103-HgR | - | - | - |

| | | | | |
|---|---|---|---|---|
| ^{a} - no band, +: thin band, ++ medium band, +++ thick band ^{b} - no visible spot, +: clear spot at OD₆₀₀ = 3.0 but weak at 0.3, ++: clear spots at both ODs. ^{c} - no fluorescence, cells almost undetectable. +: weak fluorescence. ++: medium fluorescence, readily visible. +++: strong fluorescence. ^{d} ND: not done | | | | |

Table 4 summarizes the results. Immunospots and immunofluorescence results correlate well with each other. In all CHCS3-R2 strains the CS3 pili is very well expressed. In the CHCS3-R2 (pSSVI215-cfaI) strain, as well as in the doubly integrated strains BB06 and BB07, CFA/I pili is also very well expressed. Therefore it appears that a single strain is able to support the synthesis of two heterologous ETEC pili.

### 11. Adherence of recombinant Vc/ETEC strains to Caco-2 cells

The test was performed using the human gut cells Caco-2. Caco2-cells were grown at 37°C under 5% CO₂ to near confluence in a 24-well plate in MEM medium (GIBCO BRL, Gaithersburg, USA) supplemented with 10% fetal calf serum, thus containing roughly 10⁶ Caco-2 cells per well. The recombinant strains to test, as well as the recipient strain CVD 103-HgR, were grown on CF plates for over 16 h at 37°C. A cell suspension was then made in 0.9% NaCl and the OD₆₀₀ measured. The cells were then diluted in prewarmed MEM medium to 2x 10⁶ CFU/ml. The diluted cells were then further diluted and plated onto BHI plates in duplicate to score the initial viable count (IVC). The medium was removed from the wells containing the Caco-2 cells and replaced by 0.5 ml of the diluted bacterial cells (1:1 multiplicity of infection). Each sample was run in triplicate. The plate was then centrifuged 1'000 rpm for 2 min and incubated at 37°C for 1 hour. At the end of the incubation, 10 µl of each supernatant is appropriately diluted and 100 µl of the dilutions were plated onto BHI plates in duplicate to score the supernatant viable count (SVC). The medium was removed from the wells, which were then carefully washed 3x with 0.5 ml of 0.9 % NaCl. 0.5 ml 0.9% NaCl were then added to each well, the cells were scraped with an Eppendorf tip and the cell suspension added to an Eppendorf tube. The wells were rinsed with 0.5 ml 0.9% NaCl, which was added to the Eppendorf tube. The Eppendorf tubes were subsequently vortexed at maximum speed for a full 2 min. The cell suspensions were then appropriately diluted and the dilutions were plated onto BHI plates in duplicate to score the adherent viable count (AVC). All plates were incubated for 16-20 hours at 37°C and the colonies were scored.

The adherence of the ETEC/cholera strains was determined relative to that of CVD 103-HgR by dividing the AVC for the ETEC-cholera (AVC_{EC}) by the AVC for CVD 103-HgR (AVC_{CVD}), thus producing an adherence index (AI). Since it was determined in a preliminary experiment that, in our multiplicity of infection range, the number of adherent cells is directly proportional to the inoculum size, the actual ETEC-cholera inoculum (IVC_{EC}) was divided by the actual CVD 103-HgR inoculum (IVC_{CVD}) to produce a inoculum index (II). Finally, the AI value was normalized for the inocula by dividing AI by II, thus producing the normalized adherence index (NAI). An NAI of 1 indicates an adherence identical to that of CVD 103-HgR. An NAI above or below 1 indicates higher or lower adherence than CVD 103-HgR, respectively. The data shown in table 5 indicate that the CS6 recombinant strain CHCS6-2 and CVD 103-HgR may be similarly adherent. In contrast, CHCFA/I appears to be less adherent than CVD 103-HgR, while CHCS3 is more adherent by a factor of about 1.7.

**Table 5: Adherence of pili-producing ETEC strains**

| Test pili^{a} | Experiment | R^{b} | AI^{c} | NAI^{d} | Average NAI |
|---|---|---|---|---|---|
| CFA/I | 1 | 0.27 | 0.06 | 0.22 | |
| | 2 | 1.09 | 0.74 | 0.68 | **0.40** |
| | 3 | 0.71 | 0.21 | 0.30 | |
| CS3 | 1 | 0.59 | 1.16 | 1.96 | **1.70** |
| | 2 | 0.25 | 0.36 | 1.44 | |
| CS6 | 1 | 0.22 | 0.19 | 0.84 | |
| | 2 | 1.01 | 1.2 | 1.18 | **1.03** |
| | 3 | 1.24 | 1.35 | 1.08 | |

| | | | | | |
|---|---|---|---|---|---|
| ^{a} Strains: CVD 103-HgR (reference strain), CFA/I: CHcfal-2 (pM392), CS3: CHCS3-2 (pM392), CS6: CHCS6-2 ^{b} R: viable count ratio of the pili-producing strain inoculum and the CVD 103-HgR inocu lum. ^{c} AI: adherence index: viable count ratio of the pili-producing strain adherent cells and the CVD 103-HgR adherent cells ^{d} NAI: normalized AI: AI divided by R | | | | | |

### 12. Immunofluorescence and electron microscopy

### 12.1. Immunofluorescence

To determine the immunofluorescence, the method of Lang et al, 1990 was followed as outlined in Example 10. The test strains were CHcfal-2 (pM392) expressing CFA/I pilin, CHCS3-2 (pM392) expressing CS3, and CHCS6-2 expressing CS6. The primary antibodies were anti-CFA/I, anti-CS3, and anti-CS6 rabbit polyclonal antisera which have been adsorbed against CVD 103-HgR and E. coli K12. The parent strain CVD 103-HgR served as the negative control for each antiserum.

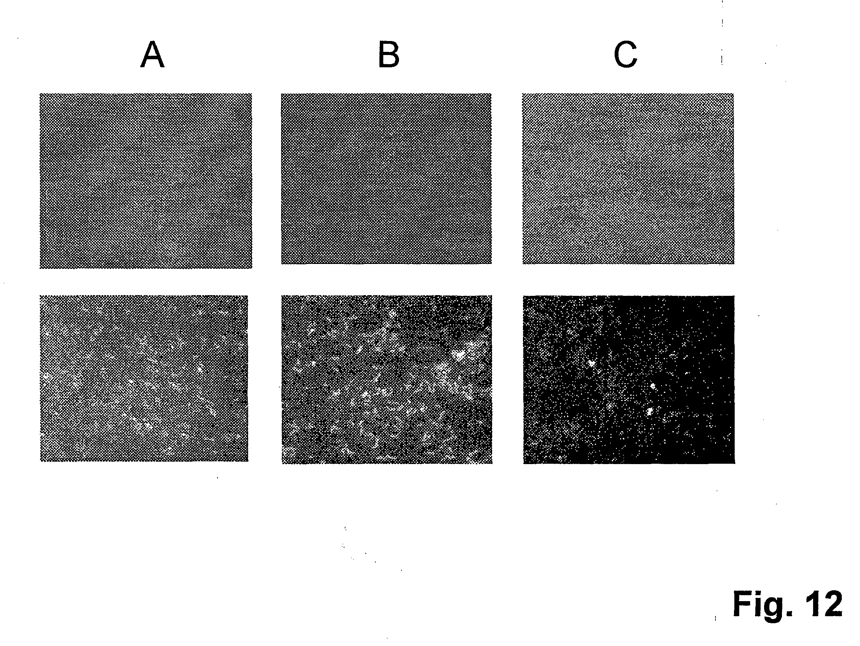

The results shown in Figure 12 indicate that for each antiserum, the negative control CVD 103-HgR does not present any fluorescence. In contrast, each of the recombinant strains incubated with the specific antiserum displays a robust fluorescence indicative of surface expression of the pili.

### 12.2 Electron microscopy

In addition to immunofluorescence, the presence of pili on the surface of the recombinant strains was studied by scanning electron microscopy. The strains tested were CHcfal-2 (pM392) and CHcfal-1 (pM392) expressing CFA/I, CHCS3-2 (pM392) expressing CS3, and CHCS6-1 and CHCS6-2 expressing CS6. The CFA/I positive controls were the ETEC strains H10407, DS7-3, and B7A for the CFA/I, CS3, and CS6 pili, respectively. CVD 103-HgR served as a negative control. All strains were grown onto CF plates at 37°C. The cultures were either fixed directly on the agar or adsorbed on glass slides, gold-covered, and poly-L-lysine treated. Following post-fixation treatment with osmium tetraoxide (OsO₄) the fixed bacteria were dried by the critical point method or by treatment by hexamethyldisilazane (HMDS). Finally, a 5 nm layer of carbon/platinum was deposed on the samples by electron beam evaporation. All samples were examined using a LEO DSM 982 scanning electron microscope.

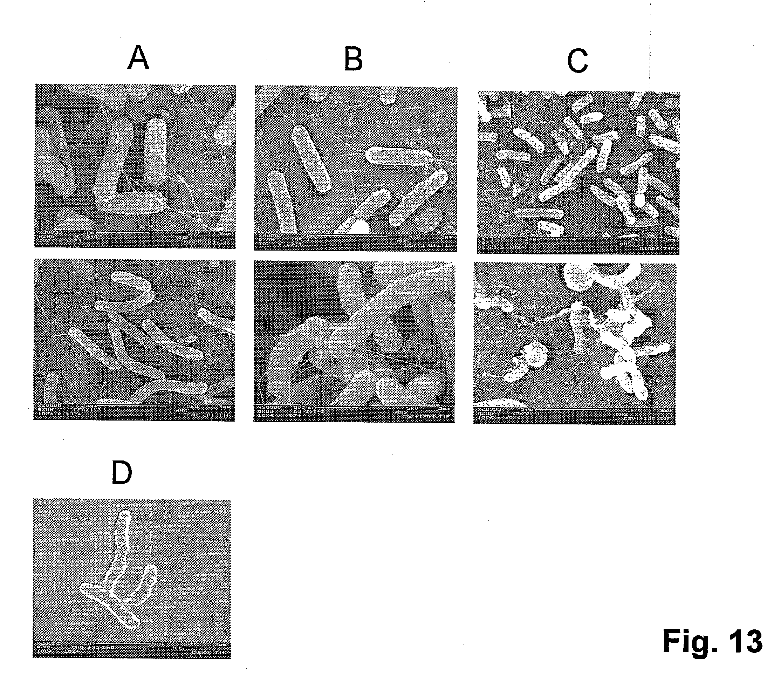

Figure 13 shows in panel A the wild-type ETEC strains H10407, expressing CFA/I pili (upper image) and the corresponding recombinant CVD 103-HgR/ETEC strain CHcfal-2 (pM392) (lower image), in panel B, the wild-type ETEC strains DS7-3, expressing CS3 pili (upper image) and the corresponding recombinant CVD 103-HgR/ETEC strain CHCS3-2 (pM392) (lower image), and in panel C, the wild-type ETEC strain B7A, expressing CS6 pili (upper image) and the corresponding recombinant CVD 103-HgR/ETEC strain CHCS6-2 (lower image). In panel D, the V. cholerae host strain CVD 103-HgR is depicted. The results show that each of the recombinant strains expresses surface pili similar to those of the corresponding wild-type ETEC strain. In contrast, CVD 103-HgR appears to have a naked surface.

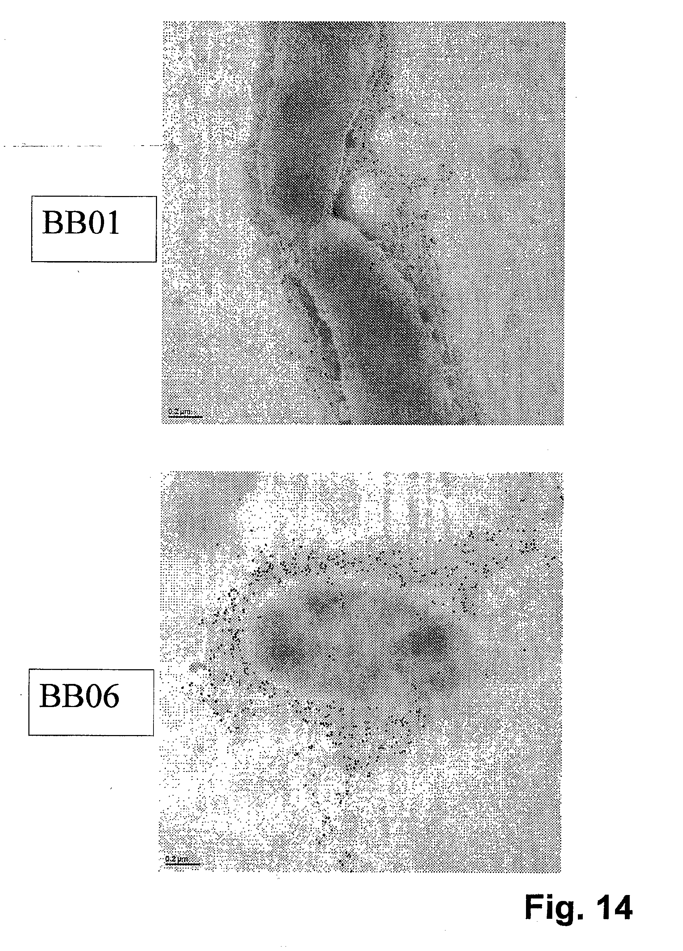

In addition, CFA/I pili expression was investigated in the monovalent CFA/I strain BB01 and in the bivalent CFA/I-CS3 strain BB06 by negative contrast electron microscopy following immunogold-labeling of the pili. Figure 14 shows that both BB01 and BB06 strains are able to efficiently synthesize CFA/I pili. In addition, it was shown by the same technique that BB06 is able to efficiently synthesize the CS3 pili.

Therefore, it is concluded from these experiments that pili are indeed synthesized on the surface of the recombinant CVD 103-HgR strain.

### 13. V. cholerae CVD 103-HgR characteristics

It is desirable that the ETEC/cholera vaccine strains conserve the same critical characteristics as the host strain CVD 103-HgR. Therefore, the following characteristics were studied: LPS pattern, toxicity, and the production of the B-subunit of cholera toxin (CT-B)

To assess the LPS pattern, overnight cultures of the various vaccine strains were grown in LBHySoy at 37°C. CVD 103-HgR was used as the control. LPS minipreparations were prepared as described (Hitchcock et al., 1983).

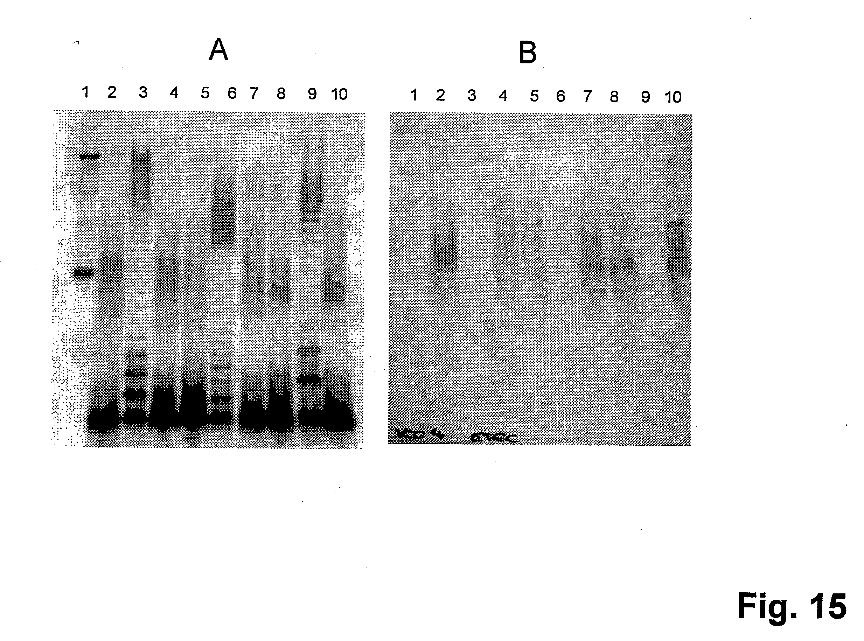

Figure 15 indicates that there is no difference between the CVD 103-HgR and the ETEC/cholera vaccine strain LPS Furthermore, the LPS pattern of the wild-type ETEC strains encoding the CFA/I, CS3, and CS6 pili is easily distinguished from that of *V*. *cholerae.* Therefore, the host strain is thereby identified as *V. cholerae* and the expression of ETEC pilin does not affect the *V. cholerae* LPS pattern.

To assess toxicity, the Y1 adrenal assay was used as described (Sack and Sack, 1975). In this test, the rounding of cells indicating cytotoxicity is qualitatively described. For the production of CT-B, the GM1-ELISA assay was used as described previously. (Svennerholm and Holmgren 1978). In this assay, the binding of CT-B to GM-1 gangliosides is detected by means of specific anti-CT-B antibodies.

**Table 6: CT-B production and cytotoxicity in recombinant strains**

| Strain | CT-B (µg /ml)^{a} | | Y1 adrenal assay^{b} |
|---|---|---|---|
| | Test 1 | Test 2 | |
| CH CS6-1 | 9.14 | nd | + |
| CH CS6-2 | 5.92 | nd | + |
| CH cfaI-1 pM392 | 1.41 | 17.45 | - |
| CH cfaI-2 pM392 | 1.23 | 12.80 | - |
| CH CS3-2 pM392 #1 | 7.89 | 13.70 | - |
| CH CS3-2 pM392 #2 | 2.78 | nd | - |
| CVD 103HgR | 8.77 | 12.49 | + |

| | | | |
|---|---|---|---|
| ^{a} The minimum value required for the production of CT-B is 2 µg /ml. ^{b}- = no rounding, + = 0-25%; ++ = 25-75%; +++ = 75%-100% | | | |

The data in table 6 indicate that the CT-B is equally well produced by CVD 103-HgR as by the recombinant strains. In the first GM1-ELISA assay some strains were unexpectedly low. However, these strains showed normal values upon retesting. Likewise, cytotoxicity of the recombinant strains and CVD 103-HgR is identically low.

In conclusion, it appears that the expression of ETEC pilin does not alter any of the critical properties of the CVD 103-HgR host strain.

### 14. Immunogenicity in mice

Groups of 5 mice were immunized subcutaneously with 100 µl of 0.9% NaCl containing 10⁹ heat-inactivated cells (60 min at 65°C) of the various recombinant strains. The negative control was the vector strain CVD 103-HgR and the positive controls were the wild-type ETEC strains. A booster immunization was administered at day 14 and the mice were sacrificed at day 29. The results were analyzed by ELISA using purified pili preparations as the coating antigens. Briefly, 2.5 microgram/ml of the specific pili was coated on a blocked ELISA plate (Nunc MAXISORP, Milian, Switzerland). After washing the wells, 100 microliters of serial dilutions of the serum to test was added and the plate incubated at room temperature for 2 hours. The plate was washed again and 100 microliters of a proxidase-labeled goat anti-mouse IgG antibody was added at a 1:2000 dilution and the plate incubated for 1 h at RT. Finally 100 microliters of a peroxidase substrate solution was add ed and the plate incubated for 10-15 min at room temperature. The results were then recorded in a plate reader (Spectramax Plus, Bucher Biotec AG, Basel, Switzerland) set at 405 nm. A significant ELISA titer is defined as being equal or superior to twice the reciprocal of the background ELISA titer. Alternatively, mice were immunized with 10⁸ live bacteria at day 1, boosted at day 14 and 28 and sacrificed on day 35.

**Table 7**

| **Strain** | **Pili tested** | **Titer (heat-killed bacteria)** | **Titer (live bacteria)** |
|---|---|---|---|
| CVD 103-HgR | CFA/I | <100 | <100 |
| H10407 | CFA/I | 6060 | 33786 |
| CHcfaI(pM392) | CFA/I | <100 | ND |
| CHcfaI-R1 | CFA/I | ND | 4468 |
| CVD 103-HgR | CFA/I | ND | <100 |
| CD79a | CFA/I | ND | 17581 |
| BB01 | CFA/I | ND | 13770 |
| CVD 103-HgR | CS3 | <100 | ND |
| DS7-3 | CS3 | 32633 | ND |
| CHCS3 (pM392) | CS3 | 9435 | ND |
| CVD 103-HgR | CS6 | <100 | <100 |
| B7A | CS6 | 1598 | 6577 |
| CHCS6-1 | CS6 | <100 | ND |
| CVD 103-HgR (pM295) | CS6 | ND | 9156 |

The results given in table 7 showed that a good immune response was obtained from strain CHCS3 (pM392). In contrast no immune response could be obtained from the CFA/I and CS6 recombinant strains. On the assumption that this may have to do with the heat inactivation of the bacteria, new groups of 5 mice were immunized with 10⁹ live bacteria followed by a with 10⁸ live bacteria booster (CFA/I) or by 10⁸ bacteria for both primary and booster immunizations (CS6). The vaccine strains CHcfaI-R1 and BB01 were used in independent experiments for the CFA/I immunizations, while strain CVD 103-HgR (pM295) was used for the CS6 immunization. In contrast to immunization with heat-killed bacteria, immunization with live bacteria led to significant immune responses from the CFA/I and CS6 recombinant strains. These experiments show that CVD 103-HgR is capable of expressing each of the CFA/I, CS3 and CS6 pili in immunogenic form on its surface.

### 15. Co-expression of three pili in the same recipient

### 15.1 Construction of trivalent strains

To demonstrate the feasibility of co-expressing three ETEC pili in one *V. cholerae* cell, the CS6-carrying plasmid pSSVI215-CS6 was electroporated in both bivalent recombinant V. cholerae strains BB06 and BB07.

The expression of the three pili operons in the resulting strains, named BB06 (pSSVI215-CS6) and BB07 (pSSVI215-CS6), was investigated by western blotting and pili ELISA. Positive controls for CS6, CS3, and CFA/I pili were the wild type strains B7A, DS7-3, and CD79a, as well as the recombinant strains CVD 103-HgR (pSSVI215-CS6), CHCS3-R2, and CHcfaI-4RI, respectively. The negative control was CVD 103-HgR.

### 15.2 Western blotting

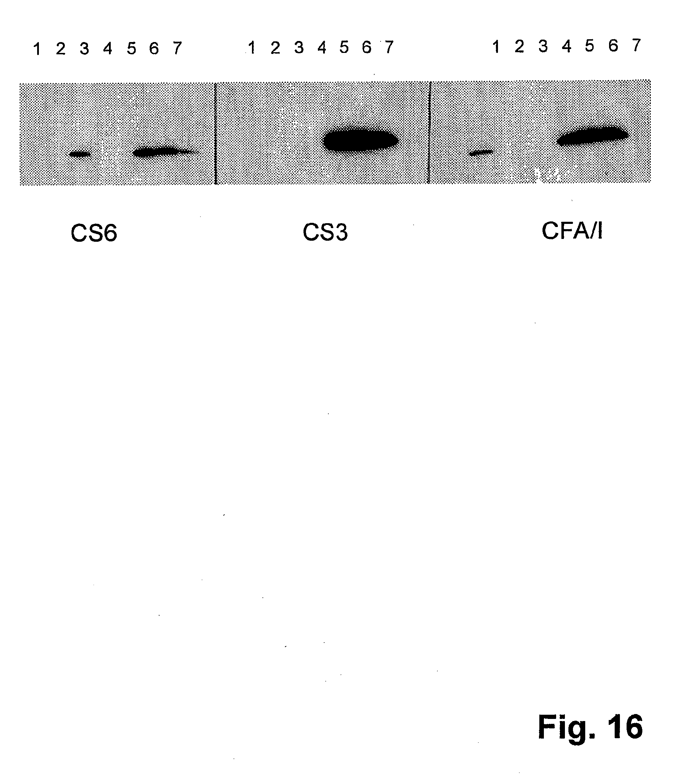

Results indicate that all three pilins are expressed in similar or even higher amounts than the wild type strain (see Figure 16). In addition, the amount of pilin displayed by bivalent or trivalent strains is equivalent to that displayed by the monovalent strains. As expected, the negative control CVD 103-HgR, as well as strains which did not contain a pili operon corresponding to the specific MAb used, did not present any bands. For unknown reasons there was only very little CS3 pilin made from the CS3-producing wild type strain DS7-3.

### 15.3 Pili ELISA

The surface expression of CFA/I, CS3, and CS6 pili in bivalent and trivalent strains was investigated by ELISA. The strains were grown in 2xCF liquid medium overnight at 37°C. The cells were then centrifuged and resuspended at an OD₆₀₀ of 0.2 in 0.9% NaCl containing 0.5% formalin. The wells of a Maxisorp plate (Nunc) were coated with 100 µl of this suspension and the plate was incubated 2 h at 37°C. The wells were then washed and blocked with a solution consisting of 0.9% NaCl containing 1% horse serum albumin (NH). 100 µl of a 1000-fold dilution of specific anti-pili monoclonal antibodies (anti-CFA/I = 2E5, anti-CS3 = 1G3, anti-CS6 = 4C9) were added to the wells and incubated 30 min at 37°C. The wells were then washed again and 100 µl of a 1000-fold diluted anti-mouse IgG horseradish peroxidase-conjugated secondary antibody was added to the wells and the plate was incubated 30 min at 37°C. The wells were washed as before and 50 µl of a peroxidase substrate (TMB, Promega Corp., Madison, USA) was added to the wells. Once the color had sufficiently developed, the reaction was stopped by 1 volume of 1M sulfuric acid and the OD₄₅₀ was measured.

**Table 8: Pili ELISA of monovalent, bivalent and trivalent ETEC pili-expressing V. cholerae strains**

| **Strain** | **CS6^{a}** | **CFA/I** | **CS3** |
|---|---|---|---|
| | | | |
| CVD 103-HgR (neg. control) | 0.008 | 0.034 | 0.017 |
| B7A (CS6 wt) | 0.172 | 0.011 | 0.010 |
| CD79a (CFA/I wt) | 0.002 | 0.972 | 0.176 |
| DS18-1 (CS3 wt) | 0.004 | 0.018 | 0.658 |
| BB01 | -0.008 | 0.624 | 0.103 |
| CHCS3-R2 | 0.012 | 0.028 | 1.069 |
| BB06 | 0.014 | 0.714 | 1.194 |
| BB07 | 0.023 | 0.876 | 1.202 |
| CVD 103-HgR (pSSVI215-CS6) | 0.090 | 0.035 | 0.008 |
| BB06 (pSSVI215-CS6) | 0.080 | 0.546 | 0.913 |
| BB07 (pSSVI215-CS6) | 0.057 | 0.478 | 0.819 |

| | | | |
|---|---|---|---|
| ^{a} ELISA titer: the values correspond to the value of the sample minus the blank. The blank corresponds to wells where PBS buffer was added instead of the monoclonal antibody. | | | |

The results indicate that both CFA/I and CS3 pili are very well surface expressed in all strains. There is a slight diminution in expression in the trivalent with respect to monovalent (BB01, CHCS3-R2) or bivalent (BB06 and BB07) strains. Surface expression of CS6 is clearly detectable, albeit at a lower level than for CFA/I and CS3. The expression of CS6 in the trivalent constellation is as good as in a monovalent form such as in CVD 103-HgR.

These results confirm the Western blots results and indicate that three ETEC pili can be surface expressed in a single *V. cholerae* cell.

### 16. Genotypic and phenotypic stability of recombinant pili operons in CVD 103-HgR

The stability of BB01 was studied after growth in 2xCFL liquid medium for more than 70 generations at 37°C. H10407 and CVD 103-HgR were used as positive and negative controls, respectively. Twenty ml 2xCFL medium was inoculated with 5-6 colonies of the tested strains and the cultures were incubated at 37°C up to stationary phase. The cultures were then diluted again in 20 ml 2xCFL medium to an OD₆₀₀ of 0.05 and incubated up to stationary phase again. Eight growth cycles were so carried out. Counting the initial growth on solid medium, a total of more than 70 generations of growth was achieved. After each growth cycle appropriate dilutions of the cultures were spread on BHI plates for viable count and colony immunoblotting. For colony immunoblotting, plates with the colonies were blotted onto nitrocellulose filters. The filters were then incubated in the presence of the CFA/I-specific MAb 2E5, followed by an alkaline phosphatase-conjugated secondary antibody incubation and detection. Table 9 shows that 100% of the BB01 colonies were positive, meaning complete genotypic and phenotypic stability. Surprisingly enough, some of the wild type bacteria lost CFA/I expression. As expected no signal was obtained from the negative control CVD 103-HgR.

In conclusion, the complete phenotypic stability of CFA/I expression in BB01 implies that both the CFA/I operon and the *rns* gene are extremely stable.

**Table 9: CFA/I stability after more than 70 generations of growth in 2xCFL medium**

| Strain | Total number of colonies | Number of negative colonies | Number of positive colonies | % positive colonies |
|---|---|---|---|---|
| BB01 | ∼600 | 0 | ∼600 | 100 |
| H10407 | 71 | 1 | 70 | 98.6 |
| CVD 103-HgR | 75 | 75 | 0 | 0 |

### Literature

1. Acheson DW et al 1993, Infect. Immun. 61 3): 1098-1104
2. Altboum Z et al 2001, Infect. Immun. 69 (5): 3150-3158
3. Altboum Z Et al. Infect Immun. 2003 71 (3): 1352-60.
4. Amann E et al. 1988, Gene 69: 301-315
5. Aubel D,et al.. Infect Immun. 1991 Apr;59 (4): 1290-9.
6. Butterton et al 1995, Infect Immun. 63: 2689-2696,
7. Butterton et al 1997, Infect Immun. 65: 2127-2138
8. Caron J et al, 1989, Proc Natl Acad Sci 86 (3): 963-967
9. Caron J et al, 1990 Infect Immun 58:3442-3444
10. Cassels FJ and MK Wolf, 1995. J.Industr.Microbiol., 15: 214-226.
11. Coster TS et al Lancet. 1995 Apr 15;345 (8955): 949-52.
12. Clemens JD et al., 1988, J Infect Dis. 1988 Aug;158 (2): 372-7
13. Cryz S. et al.1993, Infect. Immun. 61:1149-1151.;
14. Darfeuille-Michaud A et al Infect Immun. 1986 May; 52 (2): 468-75.
15. De Haan LA et al, 1991, FEMS Microbiol Lett 67 (3): 341-346
16. Dietrich G et al. Vaccine. 2003 Jan 30;21 (7-8): 678-83
17. Duthy TG,et al.. J Bacteriol. 1999 181 (18): 5847-51.
18. Favre D. and Viret JF, 2000, Biotechniques 28: 198-204
19. Froehlich BJ et al. Infect Immun. 1995 63 (12): 4849-56.
20. Gaastra W, Svennerholm AM 1996, Trends Microbiol 4 (11): 444-452
21. Gay, CC., 1971, Ann. N.Y. Acad. Sci. 176: 336-349
22. Germanier and Fürer 1975, J.Infect.Dis. 131: 553-558
23. Giron JA et al. Gene. 1997 Jun 11;192 (1): 39-43.
24. Grewal HM et al. Infect Immun. 1997 Feb;65 (2): 507-13.
25. Gustafsson B,Holme T.. J Clin Microbiol.;18 (3): 480-5.
26. Heuzenroeder MW et al FEMS Microbiol Lett. 1990 Jan 1;54 (1-3): 55-60.
27. Hitchcock P:J.and T.M. Brown, 1983, J.Bacteriol. 154: 269-277
28. Ketley et al., 1990, Infect Immun, 58: 1481-1484
29. Ketley, JM et al, 1993. FEMS Microbiol Lett 111: 15-22.
30. Kenner JR, et a1. O1. J Infect Dis. 1995 Oct; 172 (4): 1126-9.
31. Klipstein F et al. 1981. Infect. Immun. 31:144-150
32. Knutton S, Lloyd DR, McNeish AS Infect Immun. 1987 Jan; 55 (1): 86-92.
33. Lang AB et al 1990 Hum Antibodies Hybridomas.;1 (2): 96-103.
34. Levine M et al., 1988 Lancet 1988 2: 467-470
35. Levine M et al., Rev.lnfect.Dis. 1989. 11 (Suppl 3): 552-567;
36. Levine Mand Hone,1991 In Vaccine and Immunotherapy. Cryz Jr,S.J. (ed.). New York: Pergamon Press Inc, pp. 59-72).
37. Levine M and Kaper, 1993 Vaccine 11, 207-212).
38. Marron MB, Smyth CJ.. Microbiology. 1995 141 :2849-59
39. McConnell MM et al.. J Gen Microbiol. 1989 May; 135 (Pt 5): 1135-44.
40. McConnell MM et al. J Infect Dis. 1990 Feb;161 (2): 343-7.
41. McGhee and Kiyono,1993, Infect.Agents Dis.; 2: 55-73;
42. Mestecky, 1987 J. Clin. Immunol.; 7: 265-276;
43. Morgan R. L., et al., 1978 Infect. Immun. 22: 771-777
44. Nataro JP and Kaper JB., 1988. Clin Microbiol Rev. Jan; 11 (1): 142-201.
45. Peltola H et al, 1991 Lancet. ;338 (8778): 1285-9
46. Pichel M, Binsztein N, Viboud G.CS22 Infect Immun. 2000 68: 3280-5.
47. Qadri Fet al 2003 Vaccine 21: 2394-2403
48. Sack DA and Sack RB., 1975 Infect Immun. 1975 Feb; 11 (2): 334-6.
49. Sambrook (Sambrook, J., E.F. Fritsch, and T. Maniatis. 1989. In Molecular Cloning: a laboratory manual. 2nd edition. Cold Spring Harbor Laboratory, New York: Cold Spring Harbor Laboratory Press
50. Savelkoul PH et al, 1990, Microb Pathog 8 (2), 91-99
51. Schauder B et al. 1987, Gene 52 : 279-283
52. Schmidt H et al J Clin Microbiol. 1995 Mar; 33 (3): 701-5.
53. Simon RU et al, 1983. Bio/Technology 1: 784-791
54. Svennerholm A.-M. and Holmgren J., 1978. Curr. Microbiol. 1: 19-23.
55. Tacket CO, et al. J Infect Dis. 1995 Sep; 172 (3): 883-6
56. Taniguchi T et al. Infect Immun. 2001 69 (9): 5864-73.
57. Taylor DN et al. Infect Immun. 1999 Apr; 67 (4): 2030-4.
58. Valvatne H et al Infect Immun. 1996 Jul; 64 (7): 2635-42.
59. Viboud GI et al. Infect Immun. 1996 64 (4): 1233-9.
60. Walker, 1994 Vaccine 12: 387-400
61. Wolf MK, 1997 Clin Microbiol Rev. 10 (4): 569-84
62. Wu S. et al 1995 , Infect Immun, 63:4933-4938

## Claims

1. **Recombinant** *Vibrio cholera* strain comprising DNA sequences coding for at least the ETEC pili CFA/I, CS3 and CS6, wherein the DNA sequences are operationally linked to one or more promoters, and wherein the expression of one or more of said ETEC pili is enhanced by at least one ETEC regulator selected from the group consisting of Rns, CfaD, CsvR and AggR.

2. The strain according to claim 1, further comprising at least one DNA sequence coding for an ETEC pilus selected from the group consisting of CS1, CS2, CS4, CS5, CS7, CS8, CS10, CS11, CS12, CS13, CS14, CS 15, CS17, CS18, CS19, CS20, CS21, and CS22.

3. The strain according to claim 1. or 2, wherein the promoter is an ETEC promoter.

4. The strain according to claim 1, wherein said regulator is Rns.

5. The strain according to any of claims 1-4, wherein the *Vibrio cholera* strain is obtained by introducing the DNA sequences into a recipient strain selected from the group consisting of CVD103-HgR, CVD111, CVD112 and Peru-15.

6. The strain according to claim 5, wherein the recipient strain is CVD103- HgR or an equivalent strain.

7. The strain according to any of claims 1-6, wherein the DNA sequences coding for the regulator is either present on a plasmid or stably integrated into the chromosome of the recipient strain.

8. The strain according to claims 1-7, wherein the DNA sequence coding for the regulator is either present on a plasmid or stably integrated into the chromosome of the recipient strain.

9. The strain according to claim 7 or 8, wherein the DNA sequence(s) are chromosomally integrated into at least an integration site which is non-essential for inducing a protective immune response by the strain.

10. The strain according to claim 9, wherein the DNA sequence(s) are chromosomally integrated into the *hlyA, hlyB, rtxA, orfU* gene locus, or in any gene of the *ctxΦ* region.

11. The strain according to claim 10, wherein the locus of integration is *hlyB.*

12. The strain according to any of claims 1-11, wherein multiple copies of the DNA sequence(s) coding for the pili and/or regulator are integrated in tandem into a single chromosomal integration site or independently integrated into individual integration sites of the recipient chromosome.

13. The strain according to any of claims 1-12, **characterized in that** expression of the ETEC pili occurs during growth in liquid medium at a temperature between 25°C and 42°C.

14. The strain according to claim 13, **characterized in that** expression occurs during growth at a temperature of 37°C.

15. The strain according to any of claims 1-14, **characterized in that** the strain has retained the characteristics of the parent strain with respect to LPS and CT subunit B production, and cytotoxicity.

16. The strain according to any of claims 1-15, **characterized in that** the strain shows increased adherence to gut epithelial cells as compared to the recipient strain.

17. Vaccine comprising the strain according to any one of claims 1-16, optionally in a mixture with a pharmaceutically acceptable excipient and/or buffer.

18. The vaccine according to claim 17, further comprising other bacterial strains and/or toxins.

19. Use of a strain according to any of claims 1-16 for preparing a pharmaceutical composition for immunization against ETEC and/or cholera.

20. The use according to claim 19, wherein the pharmaceutical composition is for oral and/or nasal and/or rectal administration.

21. Method for producing a strain according to claims 1-16, comprising the steps of:
(a) introducing the DNA sequences coding for at least the ETEC pili CFA/I, CS3 and CS6 into a recipient strain selected from the group consisting of CVD103-HgR, CVD111, CVD112 and Peru-15, and
(b) further introducing one or more of the DNA sequence(s) coding for a regulator selected from the group consisting of Rns, CfaD, CsvR and AggR into the recipient strain.

22. Method for preparing a vaccine composition according to claim 17, comprising formulating the strain in a mixture with a pharmaceutically acceptable excipient and/or buffer.

23. Method for increasing the adherence of a *V*. *cholera* strain, comprising the steps of:
(a) introducing the DNA sequences coding for at least the ETEC pili CFA/I, CS3, and CS6 into a recipient strain selected from the group consisting of CVD103-HgR, CVD111, CVD112 and Peru-15,
(b) further introducing one or more of the DNA sequence(s) coding for a regulator selected from the group consisting of Rns, CfaD, CsvR and AggR into the recipient strain, and
(c) culturing said strain under conditions which allow surface expression of the pili.

24. Method for enhancing the surface expression of at least one ETEC pilus in a strain according to claim 1 by:
(a) introducing at least one DNA sequence encoding a regulator gene into said strain and
(b) culturing said strain under conditions which allow expression of the pilus and the regulator,
wherein the regulator gene is stably integrating into the chromosome of the strain or present on a plasmid.

## Patentansprüche

1. Rekombinanter *Vibrio cholera-*Stamm, der DNA-Sequenzen umfasst, die mindestens die ETEC-Pili CFA/I, CS3 und CS6 codieren, wobei die DNA-Sequenzen funktionell mit einem oder mehreren Promotor(en) verknüpft sind und wobei die Expression eines oder mehrerer der ETEC-Pili verstärkt ist durch mindestens einen ETEC-Regulator, der ausgewählt ist aus der Gruppe bestehend aus Rns, CfaD, CsvR und AggR.

2. Stamm nach Anspruch 1, der zudem mindestens eine DNA-Sequenz umfasst, die einen ETEC-Pilus codiert, der ausgewählt ist aus der Gruppe bestehend aus CS1, CS2, CS4, CS5, CS7, CS8, CS10, CS11, CS12, CS13, CS14, CS15, CS17, CS18, CS19, CS20, CS21 und CS22.

3. Stamm nach Anspruch 1 oder 2, wobei der Promotor ein ETEC-Promotor ist.

4. Stamm nach Anspruch 1, wobei der Regulator Rns ist.

5. Stamm nach einem der Ansprüche 1 bis 4, wobei der *Vibrio cholera*-Stamm erhalten wird durch Einbringen der DNA-Sequenzen in einen Empfängerstamm, der ausgewählt ist aus der Gruppe bestehend aus CVD103-HgR, CVD111, CVD112 und Peru-15.

6. Stamm nach Anspruch 5, wobei der Empfängerstamm CVD103-HgR oder ein äquivalenter Stamm ist.

7. Stamm nach einem der Ansprüche 1 bis 6, wobei die DNA-Sequenzen, die den Regulator codieren, entweder auf einem Plasmid vorliegen oder stabil in das Chromosom des Empfängerstammes integriert sind.

8. Stamm nach den Ansprüchen 1 bis 7, wobei die DNA-Sequenz, die den Regulator codiert, entweder auf einem Plasmid vorliegt oder stabil in das Chromosom des Empfängerstammes integriert ist.

9. Stamm nach Anspruch 7 oder 8, wobei die DNA-Sequenz(en) an mindestens einer Integrationsstelle chromosomal integriert sind, die nicht-essentiell ist für die Auslösung einer schützenden Immunantwort durch den Stamm.

10. Stamm nach Anspruch 9, wobei die DNA-Sequenz(en) in den *hlyA, hlyB, rtxA, orfU* Genlocus chromosomal integriert sind oder in ein beliebiges Gen der *ctxΦ-*Region.

11. Stamm nach Anspruch 10, wobei der Integrationslocus *hlyB* ist.

12. Stamm nach einem der Ansprüche 1 bis 11, wobei multiple Kopien der DNA-Sequenz(en), die die Pili und/oder den Regulator codieren, hintereinander an eine einzelne chromosomale Integrationsstelle integriert werden oder unabhängig an einzelne Integrationsstellen des Empfängerchromosoms integriert werden.

13. Stamm nach einem der Ansprüche 1 bis 12, **dadurch gekennzeichnet, dass** die Expression der ETEC-Pili während Wachstum in Flüssigmedium bei einer Temperatur zwischen 25°C und 42°C stattfindet.

14. Stamm nach Anspruch 13, **dadurch gekennzeichnet, dass** die Expression während Wachstum bei einer Temperatur von 37°C stattfindet.

15. Stamm nach einem der Ansprüche 1 bis 14, **dadurch gekennzeichnet, dass** der Stamm die Eigenschaften des Elternstammes bezüglich LPS- und CT-Untereinheit-B-Produktion und Cytotoxizität aufrechterhält.

16. Stamm nach einem der Ansprüche 1 bis 15, **dadurch gekennzeichnet, dass** der Stamm erhöhte Anhaftung an Darmepithelzellen im Vergleich zum Empfängerstamm aufweist.

17. Impfstoff, der den Stamm nach einem der Ansprüche 1 bis 16 umfasst, wahlweise in einem Gemisch mit einem pharmazeutisch verträglichen Exzipienten und/oder Puffer.

18. Impfstoff nach Anspruch 17, der zudem andere bakterielle Stämme und/oder Toxine umfasst.

19. Verwendung eines Stamms nach einem der Ansprüche 1 bis 16 zur Herstellung eines Arzneimittels für die Immunisierung gegen ETEC und/oder Cholera.

20. Verwendung nach Anspruch 19, wobei das Arzneimittel für die orale und/oder nasale und/oder rektale Verabreichung ist.

21. Verfahren zur Herstellung eines Stamms nach den Ansprüchen 1 bis 16, der die Schritte umfasst:
(a) das Einbringen der DNA-Sequenzen, die mindestens die ETEC-Pili CFA/I, CS3 und CS6 codieren, in einen Empfängerstamm, der ausgewählt ist aus der Gruppe bestehend aus CVD103-HgR, CVD111, CVD112 und Peru-15, und
(b) zudem das Einbringen einer oder mehrerer der DNA-Sequenz(en) in den Empfängerstamm, die einen Regulator codieren, der ausgewählt ist aus der Gruppe bestehend aus Rns, CfaD, CsvR und AggR.

22. Verfahren zur Herstellung einer Impfstoffzusammensetzung nach Anspruch 17, die das Formulieren des Stamms in ein Gemisch mit einem pharmazeutisch verträglichen Exzipienten und/oder Puffer umfasst.

23. Verfahren zur Erhöhung der Anhaftung eines *V*. cholera-Stamms, das folgende Schritte umfasst:
(a) das Einbringen der DNA-Sequenzen, die mindestens die ETEC-Pili CFA/I, CS3 und CS6 codieren, in einen Empfängerstamm, der ausgewählt ist aus der Gruppe bestehend aus CVD103-HgR, CVD111, CVD112 und Peru-15,
(b) zudem das Einbringen einer oder mehrerer DNA-Sequenz(en) in den Empfängerstamm, die einen Regulator codieren, der ausgewählt ist aus der Gruppe bestehend aus Rns, CfaD, CsvR und AggR, und
(c) das Züchten des Stamms unter Bedingungen, die eine Oberflächenexpression der Pili ermöglichen.

24. Verfahren zur Verstärkung der Oberflächenexpression von mindestens einem ETEC-Pilus in einem Stamm nach Anspruch 1 durch:
(a) das Einbringen von mindestens einer DNA-Sequenz, die ein Regulatorgen codiert, in den Stamm, und
(b) das Züchten des Stamms unter Bedingungen, die eine Expression des Pilus und des Regulators ermöglichen,
wobei das Regulatorgen stabil in das Chromosom des Stamms integriert ist oder auf einem Plasmid vorliegt.

## Revendications

1. Souche recombinante de *Vibrio cholerae* comprenant des séquences d'ADN codant pour au moins les pili bactériens d'ETEC CFA/I, CS3 et CS6, dans laquelle les séquences d'ADN sont fonctionnellement liées à un ou plusieurs promoteur(s), et dans laquelle l'expression d'un ou plusieurs desdits pili d'ETEC est améliorée par au moins un régulateur d'ETEC sélectionné parmi le groupe constitué de Rns, CfaD, CsvR et AggR.

2. Souche selon la revendication 1, comprenant en outre au moins une séquence d'ADN codant pour un pilus d'ETEC sélectionné parmi le groupe constitué de CS1, CS2, CS4, CS5, CS7, CS8, CS10, CS11, CS12, CS13, CS14, CS15, CS17, CS18, CS19, CS20, CS21 et CS22.

3. Souche selon la revendication 1 ou 2, dans laquelle le promoteur est un promoteur d'ETEC.

4. Souche selon la revendication 1, dans laquelle ledit régulateur est Rns.

5. Souche selon l'une quelconque des revendications 1 à 4, dans laquelle la souche de *Vibrio Cholerae* est obtenue par l'introduction des séquences d'ADN dans une souche receveuse sélectionnée parmi le groupe constitué de CVD103-HgR, CVD111, CVD112 et Peru-15.

6. Souche selon la revendication 5, dans laquelle la souche receveuse est CVD-103-HgR ou une souche équivalente.

7. Souche selon l'une quelconque des revendications 1 à 6, dans laquelle les séquences d'ADN codant pour le régulateur est soit présente sur un plasmide soit intégré de manière stable dans le chromosome de la souche receveuse.

8. Souche selon les revendications 1 à 7, dans laquelle la séquence d'ADN codant pour le régulateur est soit présente sur un plasmide soit intégrée de manière stable dans le chromosome de la souche receveuse.

9. Souche selon la revendication 7 ou 8, dans laquelle la(les) séquence(s) d'ADN est(sont) chromosomiquement intégrée(s) dans au moins un site d'intégration qui n'est pas essentiel pour l'induction d'une réponse immunitaire protectrice par la souche.

10. Souche selon la revendication 9, dans laquelle la(les) séquence(s) d'ADN est(sont) chromosomiquement intégrée(s) dans le locus des gènes *hlyA, hlyB, rtxA, orfU,* ou n'importe quel gène de la région *ctxΦ.*

11. Souche selon la revendication 10, dans laquelle le locus d'intégration est *hlyB.*

12. Souche selon l'une quelconque des revendications 1 à 11, dans laquelle des copies multiples de la(des) séquences d'ADN codant pour les pili et/ou le régulateur sont intégrées en tandem dans un site d'intégration chromosomique unique ou indépendamment intégrées dans des sites d'intégration individuels du chromosome receveur.

13. Souche selon l'une quelconque des revendications 1 à 12, **caractérisée en ce que** l'expression des pili d'ETEC se produit durant la croissance dans un milieu liquide a une température comprise entre 25°C et 42°C.

14. Souche selon la revendication 13, **caractérisée en ce que** l'expression se produit durant la croissance à une température de 37°C.

15. Souche selon l'une quelconque des revendications 1 à 14, **caractérisée en ce que** la souche a conservé les caractéristiques de la souche parente par rapport à la production de la sous-unité B LPS et CT, et la cytotoxicité.

16. Souche selon l'une quelconque des revendications 1 à 15, **caractérisée en ce que** la souche montre une adhérence accrue aux cellules épithéliales de l'intestin telle que comparée à la souche receveuse.

17. Vaccin comprenant la souche selon l'une quelconque des revendications 1 à 16, éventuellement dans un mélange avec un excipient et/ou un tampon pharmaceutiquement acceptable.

18. Vaccin selon la revendication 17, comprenant en outre d'autres souches bactériennes et/ou toxines.

19. Utilisation d'une souche selon l'une quelconque des revendications 1 à 16, pour la préparation d'une composition pharmaceutique pour l'immunisation contre ETEC et/ou le choléra.

20. Utilisation selon la revendication 19, dans laquelle la composition pharmaceutique sert pour l'administration par voie orale et/ou nasale et/ou rectale.

21. Procédé de production d'une souche selon les revendications 1 à 16, comprenant les étapes de :
(a) introduction des séquences d'ADN codant pour au moins les pili d'ETEC CFA/I, CS3 et CS6 dans une souche receveuse sélectionnée parmi le groupe constitué de CVD103-HgR, CVD111, CVD112 et Peru-15 et
(b) d'introduction en outre d'une ou plusieurs séquence(s) d'ADN codant pour un régulateur sélectionné parmi le groupe constitué de Rns, CfaD, CsvR et AggR dans la souche receveuse.

22. Procédé de préparation d'une composition vaccinale selon la revendication 17, comprenant la formulation de la souche dans un mélange avec un excipient et/ou un tampon pharmaceutiquement acceptable.

23. Procédé d'accroissement de l'adhérence d'une souche de *V*. *Cholerae,* comprenant les étapes de :
(a) introduction des séquences d'ADN codant pour au moins les pili d'ETEC CFA/I, CS3 et CS6 dans une souche receveuse sélectionnée parmi le groupe constitué de CVD103-HgR, CVD111, CVD112 et Peru-15,
(b) introduction supplémentaire d'une ou plusieurs séquence(s) d'ADN codant pour un régulateur sélectionné parmi le groupe constitué de Rns, CfaD, CsvR et AggR dans la souche receveuse, et
(c) de culture de ladite souche sous des conditions qui permettent l'expression de surface des pili.

24. Procédé d'amélioration de l'expression de surface d'au moins un pilus d'ETEC dans une souche selon la revendication 1 en :
(a) introduisant au moins une séquence d'ADN codant un gène régulateur dans ladite souche et
(b) cultivant ladite souche sous des conditions qui permettent l'expression du pilus et du régulateur,
dans lequel le gène régulateur s'intègre de manière stable dans le chromosome de la souche ou est présent sur un plasmide.
